# EUROPEAN PATENT SPECIFICATION

(11) **EP 2 998 001 B1**
(45) Date of publication and mention of the grant of the patent: **22.07.2020**
(21) Application number: 15192727.4
(22) Date of filing: 09.05.2013
(51) Int. Cl.: A61K 8/34, A61K 8/20, A61K 8/22, A61K 8/33, A61K 8/35, A61K 8/368, A61K 8/37, A61K 8/41, A61K 8/42, A61K 8/46, A61K 8/49, A61K 8/73, A61K 8/92, A61Q 5/02, A61Q 11/00, A61Q 11/02, A61Q 5/10, A61Q 9/02

(54) **COMPOSITION FOR REDUCTION OF TRPA1 AND TRPV1 SENSATIONS**
ZUSAMMENSETZUNG ZUR VERMINDERUNG VON TRPA1- UND TRPV1-EMPFINDUNGEN
COMPOSITION POUR LA RÉDUCTION DES SENSATIONS TRPV1 ET TRPA1

(30) Priority: 25.05.2012 US 201261652035 P; 14.08.2012 US 201261682887 P; 30.04.2013 US 201313873749
(43) Date of publication of application: 23.03.2016
(62) Divisional of application: 13722969.6
(73) Proprietor: The Procter & Gamble Company, Cincinnati, OH 45202 (US)
(72) Inventor: Haught, John Christian, Cincinnati, OH Ohio 45202 (US); Sreekrishna, Koti Tatachar, Cincinnati, OH Ohio 45202 (US); Das, Sourav, Memphis, TN Tennessee 38105 (US); Hoke, Steven Hamilton II, Cincinnati, OH Ohio 45202 (US); Coffindaffer, Timothy Woodrow, Mainville, OH Ohio 45039 (US); Glandorf, William Michael, Cincinnati, OH Ohio 45202 (US); Bakes, Katherine Anne, Cincinnati, OH Ohio 45202 (US)
(74) Representative: P&G Patent Germany

(56) References cited:
- EP-A1- 1 506 166
- WO-A1-93/17655
- DE-A1-102006 032 233
- JP-A- 2012 080 840
- US-A1- 2004 187 226
- US-A1- 2008 153 845
- US-A1- 2012 121 737
- DATABASE GNPD [Online] MINTEL; 28 February 2009 (2009-02-28), ANONYMOUS: "Whitening mouthwash", XP002752002, retrieved from www.gnpd.com Database accession no. 1051335
- J. VRIENS ET AL: "Pharmacology of Vanilloid Transient Receptor Potential Cation Channels", MOLECULAR PHARMACOLOGY, vol. 75, no. 6, 18 March 2009 (2009-03-18) , pages 1262-1279, XP055200762, ISSN: 0026-895X, DOI: 10.1124/mol.109.055624
- John. Read: "Recent Progress in the Menthone Chemistry.", Chemical Reviews, vol. 7, no. 1, 1 March 1930 (1930-03-01), pages 1-50, XP055113072, ISSN: 0009-2665, DOI: 10.1021/cr60025a001

## Description

### FIELD OF THE INVENTION

The present invention relates to a composition comprising at least 0.5% by weight of the personal care composition menthol and at least one TRPV1 receptor antagonist, and an optional TRPA1 antagonist, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; or cellulose acetate to reduce the perceived burn sensation associated with menthol.

### BACKGROUND OF THE INVENTION

In Personal Care Products, such as Oral Care Compositions the use of menthol is extensive and associated with a cleansing feeling and freshness when used at tolerable levels. Hydrogen peroxide is used as an antimicrobial, whitening agent, and in the hair coloring process. When used at high levels (>0.3% menthol or >0.1% hydrogen peroxide), they can be associated with burning and pain, as highlighted by G. Wasner et al, Brain, 127:1159-1171 (2004); K. Hill & M. Schaefer, Cell Calcium 45:155-164 (2009); and Kao in JP 2011136953.

Menthol is a well-established TRPM8 agonist that provides a chemical induced cooling response. Due to menthol's volatility, it also stimulates the olfactory bulb, providing a characteristic scent. At high levels, it can also induce a burning sensation in the nasal cavity (Renner & Schreiber, Exp. Brain Res., 217:1-14 (2012)). Further, at high levels, menthol or hydrogen peroxide are thought to activate the TRPA1 and TRPV1 receptors (P Baraldi et al, J. Med. Chem., 53:5085-5107 (2010)), which have been associated with the sensation of pain and irritation. The sensation of pain due to high levels of menthol or hydrogen peroxide can be characterized as a burning sensation or irritation when below the pain threshold (Craig & Bushnell, Science 265:252-5 (1994)); and (JP 06065044). Ahern and Matta (US Pub. No. 20110104301) attempted to modulate these receptors independent of the pain source by administering, in the presence of anesthetic, high levels of menthol among other compounds. Although menthol and peroxide activate the TRPA1 and TRPV1 receptors, they do so at only high concentrations.

As TRPA1 and TRPV1 are up-regulated by more than one agonist, a broad based blocker to each of these receptors would have the undesirable effects of losing the positive sensations that are generated from them, such as taste, tingle and astringency reduction from TRPA1 agonists and warming and astringency reduction from TRPV1 agonists. In US Pub. No. 20080153845, they illustrate TRPV1 antagonists to capsaicin, which they highlight as eliminating all taste sensations. Maintaining taste and positive sensory responses are necessary for flavor perception from Oral Care products and for scent perception from skin and hair products. WO1993/017655A1 discloses personal compositions comprising menthol together with bornyl acetate or eucalyptus oil, wherein eucalyptus oil comprises inter alia piperitone. A whitening mouthwash comprising the combination of menthol and eucalyptus oil can be found at www.gnpd.com (Database accession no. 1051335).

It was found in WO 2009087242 that the capsaicin antagonist trans-tert-butyl cyclohexanol helped to reduce negative skin sensations from cosmetic products, e.g. together with piperitone in menthol comprising compositions (US2012/121737A1). Further, due to the high level of menthol or hydrogen peroxide needed to activate TRPA1 and TRPV1, molecules that can inhibit traditional agonists to these receptors are functionally ineffective and require a molecule or combination of molecules specific to menthol or hydrogen peroxide. As a muscle soothing cream (US20090098213) high levels of menthol are delivered to generate the burning sensation, along with TRPV1 or TRPA1 agonists. Delivering high levels of menthol or hydrogen peroxide whether delivered to the mouth, skin, scalp, or hair, or without the burning sensation would be desirable as a positive signal of efficacy. Further, some of these molecules may exhibit the ability to reduce sulfur and amine species present in the body in the form of Michael Acceptors (Yoshida et al., Tetrahedron Letters, 51:5134-5136 (2010)). This effect of sulfur modification was demonstrated on the TRPA1 cysteine residues (C415S, C422S, and C622S) in response to Isothiocyanates in Mustard Oil by Macpherson et al., Nature, 445:541-545 (2007). An additional benefit of high menthol levels would be improved antimicrobial efficacy to dentifrice and rinse formulations, giving rise to formulas able to provide improved plaque and gingivitis reductions.

Therefore, what is needed is a composition and method that can reduce the negative sensations associated with menthol through activation of the TRPV1 and optional TRPA1 receptors, but that does not completely inhibit the TRPV1 and TRPA1 receptors.

### SUMMARY OF THE INVENTION

A personal care composition is provided that comprises at least 0.5% by weight of the personal care composition of menthol; and at least one of an antagonist to TRPV1 receptor, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; or cellulose acetate.

A method of reducing the negative sensations produced by the application of personal care compositions is provided that comprises providing an personal care composition having at least about 0.5% by weight of the personal care composition of menthol; at least one of an antagonist to TRPV1 receptor, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; or cellulose acetate; and contacting a body surface with the personal care composition.

### DETAILED DESCRIPTION OF THE INVENTION

It has now surprisingly been found antagonists to menthol's TRPA1 and TRPV1 response provide a noticeable reduction in the burning sensation when high levels of menthol are used. It has also been found that hydrogen peroxide acts similarly in activating the TRPA1 and TRPV1 receptors and the antagonists that shut down the menthol negative sensation also help to reduce the perceived burning/warming sensation from hydrogen peroxide. Surprisingly, these antagonists to menthol or hydrogen peroxide act specific to the TPRA1 and TRPV1 evoked sensations from menthol or hydrogen peroxide, as many of these antagonists do not block the standard agonists used on these receptors; allyl isothiocyanate which is specific to TRPA1 (does not activate TRPV1) and capsaicin which is specific to TRPV1 (does not activate TRPA1). Additionally, as menthol and hydrogen peroxide act across both the TRPA1 and TRPV1 receptors, there is a need for antagonists that block activation of both the TRPA1 and TRPV1 receptor. Therefore, there is an unmet need to provide antagonists to this burn sensation generated from the activation of TRPV1 and optional TRPA1, which is met by the present invention, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; cellulose acetate.

The negative sensorial attributes of menthol's activation of TRPA1 and TRPV1, such as burning/irritation sensation for TRPA1 and warming/burning for TRPV1, can be mitigated by combining the menthol in a personal care composition with an antagonist to menthol's activation of these receptors. Similarly, hydrogen peroxide also activates TRPA1 and TRPV1 receptors and the negative sensorial attributes associated with the activation of these receptors can be mitigated by combining the hydrogen peroxide in a personal care composition with an antagonist to hydrogen peroxide's activation of these receptors. The antagonists may be delivered with the agonist or sequenced by delivering one first and then the other via different products or applications. The present invention relates to personal care compositions and methods of using the personal care compositions containing >0.5% menthol, and which also include antagonists to the TRPV1 receptor, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; cellulose acetate.

Without being limited by theory, it is now believed that the negative sensations produced by menthol activation of TRPA1 and TRPV1 receptors can be reduced by the use of TRPA1 and TRPV1 antagonists specific to menthol activation as claimed herein.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C (i.e. room temperature) unless otherwise specified

As used herein, the word "about" means +/- 10 percent.

As used herein, the word "include," and its variants, are intended to be non-limiting, such that recitation of items in a list is not to the exclusion of other like items that may also be useful in the materials, compositions, devices, and methods of this invention.

By "personal care composition" is meant a product which in the ordinary course of usage is applied to or contacted with a body surface to provide a beneficial effect. Body surface includes skin, for example dermal or mucosal; body surface also includes structures associated with the body surface for example hair, teeth, or nails. Examples of personal care compositions include a product applied to a human body for improving appearance, cleansing, odor control or general aesthetics. Non-limiting examples of personal care compositions include hair coloring compositions, oral care compositions, after shave gels and creams, pre-shave preparations, shaving gels, creams, or foams, moisturizers and lotions, cough and cold compositions, leave-on skin lotions and creams, shampoos, conditioners, shower gels, bar soaps, toilet bars, antiperspirants, deodorants, depilatories, lipsticks, foundations, mascara, sunless tanners and sunscreen lotions.

By a "hair coloring composition" it is meant a composition suitable for changing the color of hair. The hair coloring composition can comprise oxidative precursor dyes, direct dyes or even no or substantially no dyes in case of bleaching only compositions where the change of color is mainly caused by the degradation of the natural melanin contained in the hair shaft or bleaching of artificial dyes that have been delivered by a previous coloring event, by hydrogen peroxide.

The hair coloring compositions disclosed comprise at least one source of hydrogen peroxide. Hydrogen peroxide is valuable for the initial solubilization and decolorization of the melanin (bleaching) and accelerates the oxidation of the oxidative dye precursors (oxidative dyeing) in the hair shaft. A solution of hydrogen peroxide may be used, as well as water-soluble inorganic oxidizing agents which are capable of yielding hydrogen peroxide in an aqueous solution may also be used. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. The compositions of the invention may typically comprise from about 0.1% to about 10% by weight, or from about 1% to about 7% by weight, or from about 2% to about 5% by weight of an hydrogen peroxide agent.

The hair coloring compositions disclosed may be formulated in any type of known chassis, such as a cream, a water based gel network thickener system, foam, or mousse. An exemplary gel network thickener system of this invention may be provided by a tertiary surfactant system. This system comprises a first anionic component selected from C8 to C30 alkyl phosphates, C8 to C30 alkyl ether phosphates or mixtures thereof, a second component selected from C14 to C30 fatty alcohols and a third non-ionic component selected from polyoxyethylene C14 to C30 alkyl ethers.

Those skilled in the art will recognize that gel network thickener systems usually have a complex structure of networked lamellar bi-layers and/or vesicles and sometimes crystals. These systems usually have creamy appearance and feel and are thus particularly desirable.

The hair coloring compositions disclosed may comprise in addition to the ingredients indicated above further ingredients in order to further enhance the properties of the composition, including but not limited to: solvents (e.g. glycerine); oxidative dyes, direct dyes; oxidizing agents; radical scavengers; thickeners or rheology modifiers; chelants (e.g. EDDS or DTPMP); pH modifiers and buffering agents (e.g. ammonia and ammonia source); carbonate ion sources; peroxymonocarbonate ion sources; anionic, cationic, nonionic, amphoteric or zwitterionic surfactants, or mixtures thereof; anionic, cationic, nonionic, amphoteric or zwitterionic polymers, or mixtures thereof; fragrances; enzymes; dispersing agents; peroxide stabilizing agents; antioxidants; natural ingredients, e.g. proteins and protein compounds, and plant extracts; conditioning agents including silicones and cationic polymers, ceramides, preserving agents; and opacifiers and pearling agents (such as titanium dioxide and mica). Some adjuvants referred to above, but not specifically described below, which are suitable are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions) are useful in identifying specific adjuvants to achieve a particular purpose or multipurpose. A few of these ingredients are discussed hereinbelow.

Additional examples of ingredients are listed in WO2011034868 or CA2567189, for example.

The hair coloring compositions disclosed will typically comprise water as a main ingredient, for example at least about 50%, or 60% or 70% by weight of water.

By "oral care composition", as used herein, is meant a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, tooth gel, subgingival gel, mouth rinse, mousse, foam, mouth spray, lozenge, chewable tablet, chewing gum, tooth whitening strips, floss and floss coatings, breath freshening dissolvable strips,or denture care or adhesive product. The oral care composition may also be incorporated onto strips or films for direct application or attachment to oral surfaces.

The term "dentifrice", as used herein, includes tooth or subgingival -paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

The term "dispenser", as used herein, means any pump, tube, or container suitable for dispensing compositions such as dentifrices.

The term "teeth", as used herein, refers to natural teeth as well as artificial teeth or dental prosthesis.

The term "TRPV1" or "TRPV1 receptor", as used herein, refers to the transient receptor potential vanilloid receptor 1. which is a ligand-gated, non-selective cation channel preferentially expressed on small-diameter sensory neurons and detects noxious as well as other substances.

The term "TRPV1 agonist", as used herein, refers to any compound, which at a concentration of 1 mM gives a calcium flux count of at least 1000 counts or 20% above the background level of calcium present in the cell according to the FLIPR method, as discussed herein. The term "count" is defined as the change in fluorescence of the cell lines due to the influx of calcium across the cell membrane, which reacts with the calcium sensitive dye present within the cells.

The term "TRPV1 antagonist", as used herein, refers to any component which at a concentration of 1 mM gives a reduction in calcium flux count of at least 1000 counts or 20% below the activation of TRPV1 receptor by 100 mM of hydrogen peroxide or 100 mM L-menthol of calcium present in the cell according to the FLIPR method, as discussed herein. The term "count" is defined as the change in fluorescence of the cell lines due to the influx of calcium across the cell membrane, which reacts with the calcium sensitive dye present within the cells. The antagonistic effect may also be measured by looking at lower concentrations of the receptor agonist, such as hydrogen peroxide or L-menthol at 500 µM or lower. In certain embodiments a TRPV1 receptor antagonist at a concentration of greater than 100 mM does not give a reduction of at least 20% below the maximum calcium flux count from the TRPV1 receptor activated by 350 µM capsaicin.

Disclosed TRPV1 antagonists are: (-)-Bornyl Acetate; Hydroxycitronellal; Apritone; Methyl N,N-Dimethylanthranilate; 2-Ethoxy-3-ethylpyrazine; L-Piperiton; Isobornyl Isobutyrate; 4-Acetoxy-2,5-dimethyl-3(2H)-furanone; Tripropylamine; dihydrojasmone; 1-Methyl-2-pyrole carboxaldehyde; 3-Octyl Acetate; 2-Methylbutyl isovalerate; Jasminone B; Piperonyl Isobutyrate; Phenoxyethyl Propionate; Vanillin Propylene Glycol Acetate; Octenyl Cyclopentanone; Butyl Isobutyrate; Guaiacwood Oil; Tetrahydro-4-methyl-2-(2-methyl-1 -propenyl)-2H pyran.

The term "TRPV1 enhancer", as used herein, refers to any compound that boosts the calcium flux activity of an agonist that directly activates TRPV1, but does not directly activate TRPV1.

The term "TRPA1" or "TRPA1 receptor", as used herein, refers to the transient receptor potential cation channel, subfamily A, member 1, having a large cysteine-rich N-terminus that contains 18 predicted ankyrin repeats. TRPA1 is a ligand-gated, non-selective cation channel preferentially expressed on small diameter sensory neurons.

The term "TRPA1 agonist", as used herein, refers to any compound, which at a concentration of 1 mM gives a calcium flux count of at least 1000 counts or 20% above the background level of calcium present in the cell according to the FLIPR method, as discussed herein. The term "count" is defined as the change in fluorescence of the cell lines due to the influx of calcium across the cell membrane, which reacts with the calcium sensitive dye present within the cells.

The term "TRPA1 antagonist", as used herein, refers to any component, which at a concentration of 1 mM gives a reduction in calcium flux count of at least 1000 counts or 20% below the activation of TRPA1 receptor by 100 mM of hydrogen peroxide or 100 mM L-menthol of calcium present in the cell according to the FLIPR method, as discussed herein. The term "count" is defined as the change in fluorescence of the cell lines due to the influx of calcium across the cell membrane, which reacts with the calcium sensitive dye present within the cells. The antagonistic effect may also be measured by looking at lower concentrations of the receptor agonist, such as hydrogen peroxide or L-menthol at 100 µM or lower. In certain embodiments a TRPA1 receptor antagonist at a concentration of greater than 100 mM does not give a reduction of at least 20% below the maximum calcium flux count from the TRPA1 receptor activated by 50 mM allyl isothiocyanate.

Wherein the TRPA1 antagonist may include one or more of the following: cinnamon bark oil; γ-Dodecalactone; vanillic acid; γ-Methyl Decalactone; trans, trans-2,4-Nonadienal; 4-Allyl-2,6-dimethoxyphenol; o-Methoxycinnamaldehyde; 4-Methyl-2-phenyl-2 Pentenal (mix of cis and trans); 2-Methoxy-4-propyl-phenol; Methyl 2-methoxy-benzoate; δ-Tetradecalactone; 1-Methyl-2-pyrole carboxaldehyde; 3,3,5-Trimethylcyclohexanol; N-(2-Hydroxyethyl) lactamide; 2-(3-Phenylpropyl) tetrahydrofuran; Anisyl Butyrate; Methyl-4-phenyl butyrate; 3-Heptyldihydro-5-methyl-2(3H)-furanone; 3- acetylsulfanylhexyl acetate; 3-methyl-5-propyl-2-Cyclohexen-1-one; Isobornyl Isobutyrate; Bornyl Valerate; Citronellyl acetate; (2S,5S,6S)-6-) Hydroxydihydrotheaspirane; trans-2-Hexenal.

The term "TRPA1 enhancer", as used herein, refers to any compound that boosts the calcium flux activity of an agonist that directly activates TRPA1, but does not directly activate TRPA1.

The term "Michael Acceptor", as used herein, refers to alkenes attached to electron-withdrawing groups such as esters, ketones, nitriles, and nitros, where the beta carbon is the electrophile. The addition reaction is the addition of a nucleophile to a carbanion or to another nucleophile of an α,β-unsaturated carbonyl compound.

The Michael Acceptor may have the dual functionality of chelating stain bodies and thus reducing the surface shade from a darker to a lighter color. On teeth, this may have the appearance of whitening and on skin may have the appearance of lightening.

It is desirable that oral care compositions for use in cleaning and care of the oral cavity impart a fresh and clean feeling as this provides users with a signal of continuing freshness and cleanliness. In addition to the feeling of cleanliness, users also want to experience the benefits of oral care actives like anti-tartar agents, for example, through their oral care regimen. The ability to formulate a user acceptable oral care composition, however, raises challenges as many of the components used to impart a flavor, deliver a benefit, or that are part of the base for the oral care composition, add unwanted tastes or sensations along with the targeted benefit for which they are added. Thus, formulating oral care compositions can be a balancing act between acceptable flavor and acceptable benefits.

The first group of components which reduce the burn associated with menthol or hydrogen peroxide in an oral care composition are Transient Receptor Potential Vanilloid 1 (TRPV1) antagonists. In looking at this receptor, it was discovered that combining antagonists of this receptor in the presence of the agonists menthol or hydrogen peroxide, caused a surprising effect. By adding a TRPV1 antagonist to an oral care composition with high levels of menthol or hydrogen peroxide (compositions having >0.5% menthol or >0.2% hydrogen peroxide), the user of an oral care composition experiences an improved perception as compared to an oral care composition without the TRPV1 antagonist. Thus, the TRPV1 antagonist is working to off-set the burning or warming sensation associated with menthol or hydrogen peroxide activation of TRPV1. TRPV1 responds to, for example, both noxious and painful stimuli. A noxious stimulus would include those which give a burning sensation.

The second group of components which help to reduce the burn associated with menthol or hydrogen peroxide in an oral care composition are Transient Receptor Potential Ankryin 1 (TRPA1) antagonists. In looking at this receptor, it was discovered that combining antagonists of this receptor in the presence of the agonists menthol or hydrogen peroxide, caused a surprising effect. By adding a TRPA1 antagonist to an oral care composition with high levels of menthol or hydrogen peroxide, the user of a composition experiences an improved perception as compared to an oral care composition without the TRPA1 antagonist. Thus, the TRPA1 antagonist is working to off-set the burning, irritating, or off-tasting sensation associated with menthol or hydrogen peroxide activation of TRPA1.

Further, where the agonist, such as menthol or hydrogen peroxide, targets both TRPA1 and TRV1, applying antagonists to each receptor in the same composition works synergistically to reduce the burning or negative sensation or to provide a single antagonists that hits both TRPA1 and TRPV1 provided a more desirable effect than having an antagonist to a single receptor.

In addition to the claimed TRPV1 and optional TRPA1 antagonists the oral care compositions of the present invention may include one or more of the following components, which can include metal salts, sweeteners, carrier materials, antimicrobial agents, bad breath reduction agents, bleaching agents separate from hydrogen peroxide, surfactants, flavors, anti-tartar agents, colorants, sensates, abrasive polishing materials, thickening materials, humectants, and other additives.

Actives and other ingredients may be categorized or described herein by their cosmetic benefit, therapeutic benefit, or their postulated mode of action or function. However, it is to be understood that the active and other ingredients useful herein can, in some instances, provide more than one cosmetic benefit, therapeutic benefit, function, or can operate via more than one mode of action. Therefore, classifications herein are made for the sake of convenience and are not intended to limit an ingredient to the particularly stated function(s) or activities listed.

A metal salt includes zinc salts, stannous salts, potassium salts, copper salts, alkali metal bicarbonate slats, and combinations thereof. Metal salts have a wide range of functions from antimicrobial agents to sensitivity agents or buffers. The oral care compositions of the present invention may contain metal salt in an amount from about 0.05% to about 11%, from about 0.5% to about 7%, or from about 1% to about 5%, by total weight of the oral care composition.

It is common to have a fluoride compound present in dentifrices and other oral care compositions in an amount sufficient to give a fluoride ion concentration in the composition of from about 0.0025% to about 5.0% or from about 0.005% to about 2.0%, by weight of the oral care composition to provide anticaries effectiveness. A wide variety of fluoride ion-yielding materials can be employed as sources of soluble fluoride in the present invention. Representative fluoride ion sources include: stannous fluoride, sodium fluoride, potassium fluoride, amine fluoride, sodium monofluorophosphate, indium fluoride, amine fluorides such as Olaflur, and many others. Examples of suitable fluoride ion-yielding materials are found in U.S. Pat. No. 3,535,421 to Briner et al. and U.S. Pat. No. 3,678,154 to Widder et al.

Stannous salts include stannous fluoride, stannous chloride, stannous iodide, stannous chlorofluoride, stannous actetate, stannous hexafluorozirconate, stannous sulfate, stannous lactate, stannous tartrate, stannous gluconate, stannous citrate, stannous malate, stannous glycinate, stannous pyrophosphate, stannous metaphosphate, stannous oxalate, stannous phosphate, stannous carbonate, and combinations thereof. Dentifrices containing stannous salts, particularly stannous fluoride and stannous chloride, are described in U.S. Pat. No. 5,004,597 to Majeti et al. Other descriptions of stannous salts are found in U.S. Pat. No. 5,578,293 issued to Prencipe et al. and in U.S. Pat. No. 5,281,410 issued to Lukacovic et al. In addition to the stannous ion source, other ingredients used to stabilize the stannous may be included, such as the ingredients described in Majeti et al. and Prencipe et al.

Zinc salts include zinc fluoride, zinc chloride, zinc iodide, zinc chlorofluoride, zinc actetate, zinc hexafluorozirconate, zinc sulfate, zinc lactate, zinc tartrate, zinc gluconate, zinc citrate, zinc malate, zinc glycinate, zinc pyrophosphate, zinc metaphosphate, zinc oxalate, zinc phosphate, zinc carbonate, and combinations thereof.

Potassium salts include potassium nitrate, potassium citrate, potassium oxalate, potassium bicarbonate, potassium acetate, potassium chloride, and combinations thereof.

In one embodiment, the copper salt is selected from copper fluoride, copper chloride, copper iodide, copper chlorofluoride, copper actetate, copper hexafluorozirconate, copper sulfate, copper lactate, copper tartrate, copper gluconate, copper citrate, copper malate, copper glycinate, copper pyrophosphate, copper metaphosphate, copper oxalate, copper phosphate, copper carbonate, and combinations thereof. In a further embodiment, the copper salt is selected from copper gluconate, copper acetate, copper glycinate, and combinations thereof.

Alkali metal bicarbonate salts are soluble in water and unless stabilized, tend to release carbon dioxide in an aqueous system. Sodium bicarbonate, also known as baking soda, is the preferred alkali metal bicarbonate salt. The alkali metal bicarbonate salt also functions as a buffering agent. Because of the pH at which alkali metal bicarbonate salts buffer, the bicarbonate salt may be in a phase separate from the stannous ion source. In certain embodiments, the oral care composition of the present invention may contain from about 0.5% to about 50%, from about 0.5% to about 30%, from about 2% to about 20%, or from about 5% to about 18% of an alkali metal bicarbonate salt, by weight of the oral care composition.

Some metal salts which may be used in the present invention, such as zinc chloride, zinc citrate, copper gluconate, and zinc gluconate, are also associated with an off taste described as dirty, dry, earthy, metallic, sour, bitter, and astringent. See, for example, an article by Hu, Hongzhen, et al in Nature Chemical Biology (2009), 5 (3), Pages 183-190, entitled: Zinc Activates Damage-Sensing TRPA1 Ion Channels.

Sweeteners include saccharin, chloro-sucrose (sucralose), steviolglycosides, rebaudioside A, rebaudioside B, rebaudioside C, rebaudioside D, rebaudioside E, rebaudioside F, dulcoside A, dulcoside B, rubusoside, stevia, stevioside, acesulfame K, xylitol, neohesperidine DC, alitame, aspartame, neotame, alitame, thaumatin, cyclamate, glycyrrhizin, mogroside IV, mogroside V, Luo Han Guo sweetener, siamenoside, monatin and its salts (monatin SS, RR, RS, SR), curculin, monellin, mabinlin, brazzein, hemandulcin, phyllodulcin, glycyphyllin, phloridzin, trilobatin, baiyanoside, osladin, polypodoside A, pterocaryoside A, pterocaryoside B, mukurozioside, phlomisoside I, periandrin I, abrusoside A, cyclocarioside I,N-[N-[3-(3-hydroxy-4-methoxyphenyl)propyl]-L-α-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and combinations thereof.

Rebiana is a steviolglycoside from Cargill Corp., Minneapolis, MN, which is an extract from the leaves of the Stevia rebaudiana plant (hereinafter referred to as "Rebiana"). This is a crystalline diterpene glycoside, about 300x sweeter than sucrose. Examples of suitable stevioglycosides which may be combined include rebaudioside A, rebaudioside B, rebaudioside C, rebaudioside D, rebaudioside E, rebaudioside F, dulcoside A, dulcoside B, rubusoside, stevioside, or steviolbioside. According to particularly desirable embodiments of the present invention, the combination of high-potency sweeteners comprises rebaudioside A in combination with rebaudioside B, rebaudioside C, rebaudioside F, rebaudioside F, stevioside, steviolbioside, dulcoside A. Sweeteners are generally included in an oral care composition at a level of about 0.0005% to about 2 %, by total weight of the oral care composition.

Carrier materials include water, glycerin, sorbitol, polyethylene glycols having a molecular weight of less than about 50,000, propylene glycol and other edible polyhydric alcohols, ethanol, or combinations thereof. The oral care compositions of the present invention include from about 5% to about 80%, by weight of the composition, of a carrier material. In certain embodiments, the compositions contain carrier materials in an amount of from about 10% to about 40%, by total weight of the oral care composition.

Antimicrobial agents include quaternary ammonium compounds. Those useful in the present invention include, for example, those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethoylstearyl ammonium chloride, quaternized 5-amino-1,3-bis(2-ethyl-hexyl)-5-methyl hexahydropyrimidine, benzalkonium chloride, benzethonium chloride and methyl benzethonium chloride are exemplary of typical quaternary ammonium antibacterial agents.

Other quaternary ammonium compounds include the pyridinium compounds. Examples of pyridinium quaternary ammonium compounds include bis[4-(R-amino)-1-pyridinium]alkanes as disclosed in U.S. Pat. No. 4,206,215, Jun. 3, 1980, to Bailey and cetylpyridinium and tetradecylpyridinium halide salts (i.e., chloride, bromide, fluoride and iodide).

The oral care compositions of the present invention may also include other antimicrobial agents including non-cationic antimicrobial agents such as halogenated diphenyl ethers, phenolic compounds including phenol and its homologs, mono and poly-alkyl and aromatic halophenols, resorcinol and its derivatives, xylitol, bisphenolic compounds and halogenated salicylanilides, benzoic esters, and halogenated carbanilides. Also useful antimicrobials are enzymes, including endoglycosidase, papain, dextranase, mutanase, and combinations thereof. Such agents are disclosed in U.S. Pat. No. 2,946,725, Jul. 26, 1960, to Norris et al. and in U.S. Pat. No. 4,051,234 to Gieske et al. Examples of other antimicrobial agents include chlorhexidine, and flavor oils such as thymol.

The compositions of the present invention may contain antimicrobial agents in an amount of from about 0.035% or more, from about 0.1% to about 1.5%, from about 0.045% to about 1.0%, or from about 0.05% to about 0.10%, by total weight of the oral care composition.

Examples of bad breath reduction agents include Michael Acceptors, which are antagonists of TRPA1 or TRPV1, such as dihydrojasmone and other cyclopentenones. Other agents include copper salts and carbonyl compounds such as ascorbic acid [3-oxo-L-gulofuranolactone]; cis-jasmone [3-methyl-2-(2-pentenyl-2-cyclopentenone]; 2,5-dimethyl-4-hydroxy-3(2H)-furanone; 5-ethyl-3-hydroxy-4-methyl-2(5H)-furanone; vanillin [4-hydroxy-3-methoxybenzaldehyde]; ethyl vanillin; anisaldehyde [4-methoxybenzaldehyde]; 3,4-methylenedioxybenzaldehyde; 3,4-dimethoxybenzaldehyde; 4-hydroxybenzaldehyde; 2-methoxybenzaldehyde; benzaldehyde; cinnamaldehyde [3-phenyl-2-propenal]; hexyl cinnamaldehyde; α-methyl cinnamaldehyde; ortho-methoxy cinnamaldehyde; citral; linalool; geraniol; eugenol;or combinations thereof. Without being limited by theory, it is believed some bad breath reduction agents work as "traps" by reacting with the thiol or sulfide and forming products with less odor impact. Some of these bad breath reduction agents provide an unwanted taste within an oral care composition, for example, anisaldehyde. The unwanted tastes often associated with these types of bad breath reduction agents include chemical, plastic, bitter, or sour.

The compositions of the present invention may contain bad breath reduction agents in an amount of from about 0.001% to about 4.0%, by total weight of the oral care composition.

Bleaching agents include peroxides, perborates, percarbonates, peroxyacids, persulfates, and combinations thereof. Suitable peroxide compounds include hydrogen peroxide, urea peroxide, calcium peroxide, sodium peroxide, zinc peroxide, or combinations thereof. One example of a percarbonate is sodium percarbonate. An example of a persulfate includes oxones. Some bleaching agents provide a burn sensation within an oral care composition, for example peroxides and percarbonates.

The compositions of the present invention may contain bleaching agents in an amount of from about 0.01% to about 30%, from about 0.1% to about 10%, or from about 0.5% to about 5%, by total weight of the oral care composition.

Surfactants may include anionic surfactants such as organophosphate, which include alkyl phosphates. These surface active organophosphate agents have a strong affinity for enamel surface and have sufficient surface binding propensity to desorb pellicle proteins and remain affixed to enamel surfaces. Suitable examples of organophosphate compounds include mono-, di- or triesters represented by the general structure below wherein Z1, Z2, or Z3 may be identical or different, at least one being an organic moiety, in one embodiment selected from linear or branched, alkyl or alkenyl group of from 1 to 22 carbon atoms, optionally substituted by one or more phosphate groups; alkoxylated alkyl or alkenyl, (poly)saccharide, polyol or polyether group.

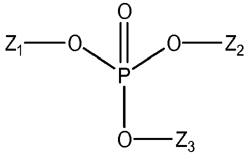

Some other organophosphate agents include alkyl or alkenyl phosphate esters represented by the following structure: 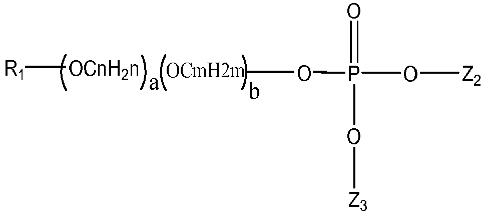 wherein R1 represents a linear or branched, alkyl or alkenyl group of from 6 to 22 carbon atoms, optionally substituted by one or more phosphate groups; n and m, are individually and separately, 2 to 4, and a and b, individually and separately, are 0 to 20; Z2 and Z3 may be identical or different, each represents hydrogen, alkali metal, ammonium, protonated alkyl amine or protonated functional alkyl amine such as an alkanolamine, or a R1-(OCnH2n)a(OCmH2m)b-group. Examples of suitable agents include alkyl and alkyl (poly)alkoxy phosphates such as lauryl phosphate; PPG5 ceteareth-10 phosphate; Laureth-1 phosphate; Laureth-3 phosphate; Laureth-9 phosphate; Trilaureth-4 phosphate; C12-18 PEG 9 phosphate; Sodium dilaureth-10 phosphate. In one embodiment, the alkyl phosphate is polymeric. Examples of polymeric alkyl phosphates include those containing repeating alkoxy groups as the polymeric portion, in particular 3 or more ethoxy, propoxy isopropoxy or butoxy groups.

Zwitterionic or amphoteric surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, such as carboxy, sulfonate, sulfate, phosphate or phosphonate. Suitable amphoteric surfactants include betaine surfactants such as disclosed in U.S. Pat. No. 5,180,577 to Polefka et al. Typical alkyl dimethyl betaines include decyl betaine or 2-(N-decyl-N,N-dimethylammonio) acetate, coco betaine or 2-(N-coco-N, N-dimethyl ammonio) acetate, myristyl betaine, palmityl betaine, lauryl betaine, cetyl betaine, stearyl betaine, etc. Amphoteric surfactants useful herein further include amine oxide surfactants. The amidobetaines are exemplified by cocoamidoethyl betaine, cocamidopropyl betaine (CAPB), and lauramidopropyl betaine. The unwanted tastes often associated with these surfactants are soapy, bitter, chemical, or artificial.

Additional suitable polymeric organophosphate agents include dextran phosphate, polyglucoside phosphate, alkyl polyglucoside phosphate, polyglyceryl phosphate, alkyl polyglyceryl phosphate, polyether phosphates and alkoxylated polyol phosphates. Some specific examples are PEG phosphate, PPG phosphate, alkyl PPG phosphate, PEG/PPG phosphate, alkyl PEG/PPG phosphate, PEG/PPG/PEG phosphate, dipropylene glycol phosphate, PEG glyceryl phosphate, PBG (polybutylene glycol) phosphate, PEG cyclodextrin phosphate, PEG sorbitan phosphate, PEG alkyl sorbitan phosphate, and PEG methyl glucoside phosphate. Suitable non-polymeric phosphates include alkyl mono glyceride phosphate, alkyl sorbitan phosphate, alkyl methyl glucoside phosphate, alkyl sucrose phosphates. The impurities in these phosphates may induce a burning sensation. Impurities may include dodecanol, dodecanal, benzaldehyde, and other TRPA1 or TRPV1 agonists.

Cationic surfactants useful in the present invention include derivatives of quaternary ammonium compounds having one long alkyl chain containing from about 8 to 18 carbon atoms such as lauryl trimethylammonium chloride, cetyl trimethylammonium bromide, coconut alkyltrimethylammonium nitrite, cetyl pyridinium fluoride, etc. Quaternary ammonium halides having detergent properties can be used, such as those described in U.S. Pat. No. 3,535,421 to Briner et al. Certain cationic surfactants can also act as germicides in the oral care compositions disclosed herein.

Examples of some flavors and flavor components that may be used in oral care compositions are mint oils, wintergreen, clove bud oil, cassia, sage, parsley oil, marjoram, lemon, orange, propenyl guaethol, heliotropine, 4-cis-heptenal, diacetyl, methyl-p-tert-butyl phenyl acetate, methyl salicylate, ethyl salicylate, 1-menthyl acetate, oxanone, α-irisone, methyl cinnamate, ethyl cinnamate, butyl cinnamate, ethyl butyrate, ethyl acetate, methyl anthranilate, iso-amyl acetate, iso-amyl butyrate, allyl caproate, eugenol, eucalyptol, thymol, cinnamic alcohol, octanol, octanal, decanol, decanal, phenylethyl alcohol, benzyl alcohol, α-terpineol, linalool, limonene, citral, neral, geranial, geraniol nerol, maltol, ethyl maltol, anethole, dihydroanethole, carvone, menthone, β-damascenone, ionone, γ-decalactone, γ-nonalactone, γ-undecalactone, or combinations thereof. Generally suitable flavoring ingredients are chemicals with structural features and functional groups that are less prone to redox reactions. These include derivatives of flavor chemicals that are saturated or contain stable aromatic rings or ester groups.

Flavors are generally present in an amount of from about 0.4 % to about 5% or from about 1% to about 3%, by total weight of the oral care composition.

Anti-tartar agents include pyrophosphate salts as a source of pyrophosphate ion. The pyrophosphate salts useful in the present compositions include, for example, the mono-, di- and tetraalkali metal pyrophosphate salts and combinations thereof. Disodium dihydrogen pyrophosphate (Na2H2P2O7), sodium acid pyrophosphate, tetrasodium pyrophosphate (Na4P2O7), and tetrapotassium pyrophosphate (K4P2O7) in their unhydrated as well as hydrated forms are further species. In compositions of the present invention, the pyrophosphate salt may be present in one of three ways: predominately dissolved, predominately undissolved, or a combination of dissolved and undissolved pyrophosphate. The amount of pyrophosphate salt useful in making these compositions is any tartar control effective amount. In varying embodiments, the amount of pyrophosphate salt may be from about 1.5% to about 15%, from about 2% to about 10%, or about 3% to about 8%, by total weight of the oral care composition.

Examples of some colorants that may be used in oral care compositions include D&C Yellow No. 10, FD&C Blue No. 1, FD&C Red No. 40, D&C Red No. 33 and combinations thereof. In certain embodiments, the composition comprises colorant in an amount of from about 0.0001 % to about 0.1% or from about 0.001% to about 0.01%, by weight of the oral care composition. Some colorants provide an unwanted taste, for example, D&C Red No. 33. The unwanted tastes often associated with this colorant are metallic, sharp, or chemical. Colorants are generally present in an amount of from about 0.001% to about 0.5%, by weight of the oral care composition.

Sensates may also be part of an oral care composition. Sensate molecules such as cooling, warming, and tingling agents are useful to deliver signals to the user. Sensates are generally present in an amount of from about 0.001% to about 0.8%, by weight of the oral care composition. The most well-known cooling sensate compound is menthol, particularly L-menthol, which is found naturally in peppermint oil notably of Mentha arvensis L and Mentha viridis L. Other isomers of menthol (neomenthol, isomenthol and neoisomenthol) have somewhat similar, but not identical odor and taste, for instance having disagreeable odor and taste described as earthy, camphor, musty, etc. The biggest difference among the isomers is in their cooling potency. L-menthol provides the most potent cooling, by having the lowest cooling threshold of about 800 ppb, which is the concentration level where the cooling effect can be clearly recognized. At this level, there is no cooling effect for the other isomers. For example, d-neomenthol is reported to have a cooling threshold of about 25,000 ppb and 1-neomenthol about 3,000 ppb. [R. Emberger and R. Hopp, "Synthesis and Sensory Characterization of Menthol Enantiomers and Their Derivatives for the Use in Nature Identical Peppermint Oils," Specialty Chemicals (1987), 7(3), 193-201].

Of the menthol isomers the 1-isomer occurs most widely in nature and is typically what is referred by the name menthol having coolant properties. L-menthol has the characteristic peppermint odor, has a clean fresh taste and exerts a cooling sensation when applied to the skin and mucosal surfaces.

Among synthetic coolants, many are derivatives of or are structurally related to menthol, for example containing the cyclohexane moiety, and derivatized with functional groups including carboxamide, ketal, ester, ether and alcohol. Examples include the p-menthanecarboxamide compounds such as N-ethyl-p-menthan-3-carboxamide, known commercially as "WS-3", and others in the series such as WS-5 (N-ethoxycarbonylmethyl-p-menthan-3-carboxamide), WS-12 (1R*,2S*)-N-(4-Methoxyphenyl)-5-methyl-2-(1-methylethyl)cyclohexanecarboxamide] and WS-14 (N-tert-butyl-p-menthan-3-carboxamide). Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional examples of synthetic coolants include alcohol derivatives such as 3-(1-menthoxy)-propane-1,2-diol known as TK-10, isopulegol (under the tradename Coolact P) and p-menthane-3,8-diol (under the tradename Coolact 38D) all available from Takasago Corp., Tokyo, Japan; menthone glycerol acetal known as MGA; menthyl esters such as menthyl acetate, menthyl acetoacetate, menthyl lactate known as Frescolat® supplied by Symrise AG, Holzminden, Germany, and monomenthyl succinate under the tradename Physcool from V. Mane FILS, Notre Dame, France. TK-10 is described in U.S. Pat. No. 4,459,425 to Amano et al. Other alcohol and ether derivatives of menthol are described in GB 1,315,626 and in U.S. Pat. No's 4,029,759; 5,608,119; and 6,956,139. WS-3 and other carboxamide cooling agents are described in U.S. Pat. No's 4,136,163; 4,150,052; 4,153,679; 4,157,384; 4,178,459 and 4,230,688.

Additional N-substituted p-menthane carboxamides are described in WO 2005/049553A1 including N-(4-cyanomethylphenyl)-p-menthanecarboxamide, N-(4-sulfamoylphenyl)-p-menthanecarboxamide, N-(4-cyanophenyl)p-menthanecarboxamide, N-(4-acetylphenyl)-p-menthanecarboxamide, N-(4-hydroxymethylphenyl)-p-menthanecarboxamide and N-(3-hydroxy-4-methoxyphenyl)-p-menthanecarboxamide. Other N-substituted p-menthane carboxamides include amino acid derivatives such as those disclosed in WO 2006/103401 and in U.S. Pat. Nos. 4,136,163; 4,178,459 and 7,189,760 such as N-((5-methyl-2-(1-methylethyl)cyclohexyl)carbonyl)glycine ethyl ester and N-((5-methyl-2-(1-methylethyl)cyclohexyl)carbonyl)alanine ethyl ester. Menthyl esters including those of amino acids such as glycine and alanine are disclosed e.g., in EP 310,299 and in U.S. Pat. Nos. 3,111,127; 3,917,613; 3,991,178; 5,5703,123; 5,725,865; 5,843,466; 6,365,215; 6,451,844; and 6,884,903. Ketal derivatives are described, e.g., in U.S. Pat. Nos. 5,266,592; 5,977,166; and 5,451,404. Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112. Reviews on the coolant activity of menthol and synthetic coolants include H. R. Watson, et al. J. Soc. Cosmet. Chem. (1978), 29, 185-200 and R. Eccles, J. Pharm. Pharmacol., (1994), 46, 618-630.

Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112 and phosphine oxides as reported in U.S. Pat. No. 4,070,496.

Some examples of warming sensates include ethanol; capsicum; nicotinate esters, such as benzyl nicotinate; polyhydric alcohols; capsicum powder; a capsicum tincture; capsicum extract; capsaicin; homocapsaicin; homodihydrocapsaicin; nonanoyl vanillyl amide; nonanoic acid vanillyl ether; vanillyl alcohol alkyl ether derivatives such as vanillyl ethyl ether, vanillyl butyl ether, vanillyl pentyl ether, and vanillyl hexyl ether; isovanillyl alcohol alkyl ethers; ethylvanillyl alcohol alkyl ethers; veratryl alcohol derivatives; substituted benzyl alcohol derivatives; substituted benzyl alcohol alkyl ethers; vanillin propylene glycol acetal; ethylvanillin propylene glycol acetal; ginger extract; ginger oil; gingerol; zingerone; or combinations thereof. Warming sensates are generally included in an oral care composition at a level of about 0.05% to about 2%, by weight of the oral care composition.

Abrasive polishing material can be any material that does not excessively abrade dentin. The oral care compositions of the present invention may comprise abrasive polishing material in an amount of from about 6% to about 70% or from about 10% to about 50%, by weight of the oral care composition. Typical abrasive polishing materials include silicas including gels and precipitates; aluminas; phosphates including orthophosphates, polymetaphosphates, and pyrophosphates; and mixtures thereof. Specific examples include dicalcium orthophosphate dihydrate, calcium pyrophosphate, tricalcium phosphate, calcium polymetaphosphate, insoluble sodium polymetaphosphate, rice hull silica, hydrated alumina, beta calcium pyrophosphate, calcium carbonate, and resinous abrasive materials such as particulate condensation products of urea and formaldehyde, and others such as disclosed by Cooley et al in U.S. Pat. No. 3,070,510. In certain embodiments, if the oral composition or particular phase comprises a polyphosphate having an average chain length of about 4 or more, calcium containing abrasives and alumina are not preferred abrasives.

Silica dental abrasives of various types are often used in oral care compositions due to their exceptional dental cleaning and polishing performance without unduly abrading tooth enamel or dentine. Silica abrasive polishing materials that may be used in the present invention, as well as other abrasives, generally have an average particle size ranging between about 0.1 to about 30 µm or from about 5 to about 15 µm. The abrasive can be precipitated silica or silica gels such as the silica xerogels described in Pader et al., U.S. Pat. No. 3,538,230 and DiGiulio, U.S. Pat. No. 3,862,307. Silica xerogels marketed under the trade name "Syloid" by the W.R. Grace & Company, Davison Chemical Division, Augusta, GA may be used. Also precipitated silica materials such as those marketed by the J. M. Huber Corporation, Edison, NJ under the trade name, "Zeodent", particularly the silica carrying the designation "Zeodent 119", may be used. The types of silica dental abrasives useful in the oral care compositions of the present invention are described in more detail in Wason, U.S. Pat. No. 4,340,583; and Rice U.S. Pat. No's 5,589,160; 5,603,920; 5,651,958; 5,658,553; and 5,716,601.

Thickening material or binders may be used to provide a desirable consistency to the oral care compositions of the present invention. For example when the oral care compositions are in the form of dentifrices, topical oral gels, mouthrinse, denture product, mouthsprays, lozenges, oral tablets or chewing gums, the amount and type of the thickening material will depend upon the form of the product. Thickening materials include carboxyvinyl polymers, carrageenan, hydroxyethyl cellulose, and water soluble salts of cellulose ethers such as sodium carboxymethylcellulose and sodium hydroxyethyl cellulose. Natural gums such as gum karaya, xanthan gum, gum arabic, and gum tragacanth can also be used. Colloidal magnesium aluminum silicate or finely divided silica can be used as part of the thickening material to further improve texture. Thickening materials can be used in an amount from about 0.1% to about 15%, by weight of the oral care composition.

Humectants keep oral care compositions from hardening upon exposure to air and certain humectants can also impart desirable sweetness of flavor to dentifrice compositions. Suitable humectants for use in the present invention include glycerin, sorbitol, polyethylene glycol, propylene glycol, xylitol, and other edible polyhydric alcohols. The oral care compositions of the present invention may comprise humectants in an amount of from about 0% to about 70% or from about 15% to about 55%, by weight of the oral care composition.

### EXAMPLES

All examples not falling within the scope of the claims are to be regarded as comparative data.

For EXAMPLES 1, 2 and 3, the first group of components which will help to reduce the burn associated with menthol or hydrogen peroxide in an oral care composition are Transient Receptor Potential Ankryin 1 (TRPA1) antagonists. In looking at this receptor, it was discovered that combining antagonists of this receptor in the presence of the agonists menthol or hydrogen peroxide, caused a surprising effect. By adding a TRPA1 antagonist to an oral care composition with high menthol levels or hydrogen peroxide, the user of the composition experienced an improved perception and in use experience of the composition, over an oral care composition without the TRPA1 antagonist. Thus, the TRPA1 antagonist is working to off-set the burning, irritating, or off-tasting sensation associated with menthol or hydrogen peroxide activation of TRPA1.

In order to determine whether TRPA1 is activated, the intracellular calcium ion (Ca²⁺) level from transfected cells with the TRPA1 receptor gene was measured. HEK-2 cells stably transfected with human TRPA1 were grown in 15 ml growth medium [high glucose DMEM (Dulbecco's Modification of Eagle's Medium) supplemented with 10% FBS (fetal bovine serum), 100µg/ml Penicillin/streptomycin, 100 µg/ml G418] in a 75 Cm² flask for 3 days at 37°C in a mammalian cell culture incubator set at 5% CO₂. Cells were detached with addition of 10 ml of PBS (phosphate buffered saline) by hand shaking gently and transferred to a 50 ml tube and centrifuged at 850 rpm for 3 minutes to remove PBS. After centrifugation, a pellet of cells was formed in the bottom of the tube separating them from the supernatant solution. The supernatant was discarded and the cell pellet suspended in 1 ml of fresh growth medium to which 5 µl (12.5 µg) of Fluo-4 AM (Molecular Probes, Inc., Eugene, OR) calcium indicator was added and incubated for 30 minutes with gentle shaking. Fluo-4 is a fluorescent dye used for quantifying cellular Ca²⁺ concentrations in the 100 nM to 1 µM range. At the end of the 30 minutes, 45 ml of assay buffer [1xHBSS (Hank's Balanced Salt Solution), 20 mM HEPES (4-(2-Hydroxyethyl)-1-piperazineethanesulfonic acid)] was added to wash the cells and the resulting combination was then centrifuged at 850 rpm for 3 minutes to remove excess buffer and Fluo-4 AM calcium indicator.

The pelleted cells were re-suspended in 10 ml assay buffer and 90 µl aliquots (∼50,000 cells) per well delivered to a 96-well assay plate containing 10 µl of test compounds (1 mM in assay buffer, final concentration 100 µM) or buffer control and incubated at room temperature for 30 minutes. After 30 minutes, the plate was placed into a fluorometric imaging plate reader (FLIPR384 from Molecular Devices) and basal fluorescence recorded (excitation wave length 488 nm and emission wave length 510 nm). Then 20 µl of the molecule being tested as a TRPA1 antagonist was added and fluorescence recorded. For determining the direct effect of test compounds on TRPA1, fluorescence was measured immediately after addition of each compound.

To determine if a molecule was an antagonist of TRPA1 receptor activation and the level of antagonism, a molecule that had >20% reduction in calcium flux compared to the menthol or hydrogen peroxide activated TRPA1 receptor was viewed as a potential antagonist.

To determine if a compound was an antagonist or a desensitizer the direct effect of a test compound was determined. For determining the direct effect of test compounds on TRPA1 receptor activation, 100 µl aliquots (∼50,000 cells) of cells prepared as described above were delivered to a 96-well assay plate and basal fluorescence recorded as noted above. Then 20 µl of the compound being tested as a TRPA1 activator was added and fluorescence recorded. If any increase in fluorescence over background was noted, then the compound was considered an agonist. The agonist activity was expressed relative to that observed with a benchmark agonist such as 50 µM allyl isothiocyanate for TRPA1 or for the purpose of this invention, L-menthol or hydrogen peroxide. If a compound did not show any agonistic activity when directly added, but inhibited activation by a known TRPA1 agonist in the preincubation study, then it was called an antagonist. If the compound showed agonist activity and caused decrease in activation by a known TRPA1 agonist in the preincubation study, then it was called a desensitizer. Additional discussion of the FLIPR method can be found in Smart et al., Characterization using FLIPR of human vanilloid VR1 receptor pharmacology, European Journal of Pharmacology 417, 51-58 (2001) and Liu et al., Development and validation of a platelet calcium flux assay using a fluorescent imaging plate reader, Analytical Biochemistry 357, 216-224 (2006).

For EXAMPLES 1, 4 and 5, the second group of components tested for their ability to reduce the burn associated with menthol or hydrogen peroxide in an oral care composition are Transient Receptor Potential Vanilloid 1 (TRPV1) antagonists. In looking at this receptor, it was discovered that combining antagonists of this receptor in the presence of the agonists menthol or hydrogen peroxide, caused a surprising effect. By adding a TRPV1 antagonist to an oral care composition with high menthol levels or hydrogen peroxide, the user of the composition experienced an improved perception and in use experience of the composition over an oral care composition without the TRPV1 antagonist. Thus, the TRPV1 antagonist is working to off-set the burning or warming sensation associated with menthol or hydrogen peroxide activation of TRPV1.

To determine whether TRPV1 was activated, the intracellular calcium ion (Ca⁺²) levels from cells transfected with the TRPV1 receptor gene was measured. HEK-239 cells stably transfected with human TRPV1 were grown in 15 ml growth medium [high glucose DMEM (Dulbecco's Modification of Eagle's Medium) supplemented with 10% FBS (fetal bovine serum), 100µg/ml Penicillin/streptomycin, 100 µg/ml G418] in a 75 Cm2 flask for 3 days at 33°C in a mammalian cell culture incubator set at 5% CO₂. Cells were detached with addition of 10 ml of PBS (phosphate buffered saline) by hand shaking gently. Cells were transferred to a 50 ml tube and centrifuged at 850 rpm for 3 minutes to remove PBS. After centrifugation, a pellet of cells formed in the bottom of the tube separating them from the supernatant solution. The supernatant was discarded and the cell pellet suspended in 1 ml of fresh growth medium to which 5 µl (12.5 µg) of Fluo-4 AM (Molecular Probes, Inc., Eugene, OR) calcium indicator was added and incubated for 30 minutes with gentle shaking. Fluo-4 is a fluorescent dye used for quantifying cellular Ca²⁺ concentrations in the 100 nM to 1 µM range. At the end of the 30 minutes, 45 ml of assay buffer [1xHBSS (Hank's Balanced Salt Solution), 20 mM HEPES (4-(2-Hydroxyethyl)-1-piperazineethanesulfonic acid)] was added to wash the cells and the resulting combination was then centrifuged at 850 rpm for 3 minutes to remove excess buffer and Fluo-4 AM calcium indicator.

The pelleted cells were re-suspended in 10 ml assay buffer and 90 µl aliquots (∼50,000 cells) per well delivered to a 96-well assay plate containing 10 µl of test compounds (1 mM in assay buffer, final concentration 100 µM) or buffer control and incubated at room temperature for 30 minutes. After 30 minutes, the plate was placed into a fluorometric imaging plate reader (FLIPR384 from Molecular Devices) and basal fluorescence recorded (excitation wave length 488 nm and emission wave length 510 nm). Then 20 µl of the compound being tested as a TRPV1 receptor activator was added and fluorescence recorded. The observed value with compound pretreated cells was compared with buffer control, with the difference between the two indicating a measure of effect of the test compound on the activator. It may be no difference (no effect), or negative (means antagonist or desensitizer) or positive (enhancer, also known as positive allosteric modulator). A molecule that had >20% reduction in calcium flux compared to the menthol or hydrogen peroxide activated TRPV1 receptor was viewed as a potential antagonist or a desensitizer. To determine if a compound was an antagonist or a desensitizer, the direct effect of a test compound was determined. For determining the direct effect of test compounds on TRPV1, 100 µl aliquots (∼50,000 cells) of cells prepared as described above were delivered to a 96-well assay plate and basal fluorescence recorded as noted above. Then 20 µl of the compound being tested as a TRPV1 receptor activator was added and fluorescence recorded. If any increase in fluorescence over background was noted, then the compound was considered an agonist. The agonist activity was expressed relative to that observed with a benchmark agonist such as 350 nM Capsaicin for TRPV1. If a compound did not show any agonistic activity when directly added, but inhibited activation by a known TRPV1 agonist in the preincubation study, then it was called an antagonist. If the compound showed agonist activity and caused decrease in activation by a known TRPV1 agonist in the preincubation study, then it was called a desensitizer. Additional discussion of the FLIPR method can be found in Smart et al., Characterization using FLIPR of human vanilloid VR1 receptor pharmacology, European Journal of Pharmacology 417, 51-58 (2001) and Liu et al., Development and validation of a platelet calcium flux assay using a fluorescent imaging plate reader, Analytical Biochemistry 357, 216-224 (2006).

The TRPV1 receptor responds to, for example, both noxious and painful stimuli. A noxious stimulus would include those which give a burning (i.e. hot) sensation.

### EXAMPLE 1

TABLE 1 below shows percent hydrogen peroxide activation (as measured by intracellular Ca²⁺ levels) of TRPV1 and TRPA1 receptors, as compared to the level of receptor activation of the control agonists (AITC for TRPA1 and capsaicin for TRPV1) normally used to test for TRPV1 and TRPA1 receptor activation. Table 1 showed the concentrations at which hydrogen peroxide's agonist activity was comparable to the control agonists on the TRPA1 and TRPV1 receptors. AITC was the control agonist for TRPA1 and capsaicin was the control for TRPV1.

**TABLE 1**

| H₂O₂ | TRPA1 | TRPV1 |
|---|---|---|
| 100 mM | 296.7% | 209.29% |
| 10 mM | 101.3% | 62.81% |
| 1mM | 123.4% | 41.13% |
| 500 µM | 129.1% | 30.21% |
| 200 µM | 111.7% | 21.18% |
| 100 µM | 91.4% | 17.45% |
| 10 µM | 9.6% | 3.10% |
| 1 µM | 1.4% | 1.51% |
| **Controls** | Ca²⁺ Count | Ca²⁺ Count |
| 50 µM AITC | 12219 | na |
| 350 nM Capsaicin | na | 16204 |

### EXAMPLE 2

The compounds listed in TABLE 2 below have been found to be antagonists of the TRPA1 receptor, in that they reduce the level of TRPA1 receptor activation when activated by hydrogen peroxide. 100 µM of antagonist was tested against 200 µM hydrogen peroxide to determine the level of reduced TRPA1 receptor activation by hydrogen peroxide. AITC was tested to demonstrate its higher level of activation (higher average Ca²⁺ count) as compared to H₂O₂.

**TABLE 2**

| Compounds tested at 100 gM (Set1, TRPA1 assay) | Average Ca⁺⁺ Counts | % Reduction of Hydrogen Peroxide TRPA1 Activation |
|---|---|---|
| Control -H2O2 (200 µM) | 7043.6 | 0.0 |
| AITC (50 µM) | 11209.0 | 0.0 |
| phloretin | 215.0 | 96.9 |
| copper(i) iodide | 678.0 | 90.4 |
| 3-mercapto-2-pentanone | 1255.5 | 82.2 |
| 1,2-propanedithiol | 2194.5 | 68.8 |
| ethyl methyl beta-phenylethyl carbinol | 2253.5 | 68.0 |
| 2-methylbutyl 2-methylbutyrate | 2685.5 | 61.9 |
| fenchone | 2761.5 | 60.8 |
| piperazine | 2815.5 | 60.0 |
| isoamyl pyruvate | 2824.0 | 59.9 |
| acetic acid isopropenyl ester | 2860.5 | 59.4 |
| isopropyl hexanoate | 2862.0 | 59.4 |
| manganese chloride | 2872.5 | 59.2 |
| 2-acetyl-5-methylfuran | 2937.0 | 58.3 |
| 3-acetylpyridine, 98% | 2981.0 | 57.7 |
| n-butyl alcohol | 3009.0 | 57.3 |
| 2-undecanol | 3049.0 | 56.7 |
| desoxycholic acid | 3058.5 | 56.6 |
| 2-nonanol | 3064.5 | 56.5 |
| 2-isobutyl-3 -methoxypyrazine | 3067.5 | 56.4 |
| gamma-terpinene | 3124.0 | 55.6 |
| 1-stearoyl-rac-glycerol | 3131.0 | 55.5 |
| amyl alcohol | 3149.0 | 55.3 |
| 2-methyl-3 -ethoxypyrazine | 3197.0 | 54.6 |
| sodium erythorbate | 3204.5 | 54.5 |
| 2-methyl-1-butanethiol | 3208.0 | 54.5 |
| 1-octen-3-yl acetate | 3228.5 | 54.2 |
| dl-verbenone | 3231.0 | 54.1 |
| 4'-methylacetophenone | 3244.5 | 53.9 |
| 3-methyl-1,2-cyclopentanedione | 3246.5 | 53.9 |
| 3-hydroxybenzoic acid | 3284.5 | 53.4 |
| ferric chloride | 3287.0 | 53.3 |
| phenylethyl isovalerate | 3291.5 | 53.3 |
| ethyl acetoacetate ethylene ketal | 3298.5 | 53.2 |
| 6-undecanone | 3357.5 | 52.3 |
| methyl linoleate | 3363.5 | 52.2 |
| alpha,alpha-dimethylhydrocinnamyl acetate | 3367.0 | 52.2 |
| 2-hexyl-4-methyl-1,3-dioxolan | 3376.0 | 52.1 |
| 3-hexanone | 3381.5 | 52.0 |
| isobutyl isobutyrate | 3383.0 | 52.0 |
| isobutyric acid isopropyl ester | 3392.5 | 51.8 |
| 1-undecanol | 3396.0 | 51.8 |
| 4-hydroxybenzaldehyde | 3424.5 | 51.4 |
| 2-hydroxy-4-methylbenzaldehyde | 3429.0 | 51.3 |
| quinoline | 3440.0 | 51.2 |
| allyl cyclohexanepropionate | 3443.0 | 51.1 |
| carvacrol | 3464.5 | 50.8 |
| p-cymen-8-ol | 3476.5 | 50.6 |
| Compounds tested at 100 µM (Set2, TRPA1 assay) | | |
| H₂O₂ (200 µM) | 8721.8 | |
| AITC (50 µM) | 14138.2 | |
| 2-phenyl-2-butenal | 3809.0 | 56.3 |
| alpha,p-dimethylstyrene | 3735.5 | 57.2 |
| isopentyl alcohol | 3778.0 | 56.7 |
| 2-methyl-1,3-dithiolane | 2402.0 | 72.5 |
| methyl benzoate | 3817.0 | 56.2 |
| Compounds tested at 100 µM (Set3, TRPA1 assay) | | |
| H₂O₂ (200 µM) | 10592.2 | |
| AITC (50 µM) | 15213.0 | |
| 2-methyl-3-heptanone | 4994.0 | 52.9 |
| 3,7-dimethyl-1-octanol, 95% | 5168.0 | 51.2 |
| butyl 4-hydroxybenzoate | 411.0 | 96.1 |
| 2,5-dimethyl-1,4-dithiane-2,5-diol | 2420.0 | 77.2 |
| 2,4,6-trithiaheptane | 4610.5 | 56.5 |
| phenylacetaldehyde | 2578.5 | 75.7 |
| benzyl cinnamate | 4828.5 | 54.4 |
| 2-hydroxy-3-methyl-2-cyclopenten-1-one hydrate | 4172.0 | 60.6 |
| 2,3-butanedithiol | 1280.5 | 87.9 |
| phenyl salicylate | 2893.5 | 72.7 |
| 5-methylhexanoic acid | 4747.0 | 55.2 |
| 2-pyrazinylethanethiol | 1987.5 | 81.2 |
| 2-chloroacetophenone | 1199.5 | 88.7 |
| acetoin | 4341.5 | 59.0 |
| 2-propionylthiazole | 4875.0 | 54.0 |
| benzothiazole | 5513.0 | 48.0 |
| butyl lactate | 5157.0 | 51.3 |
| 2-ethyl-1-hexanol | 4960.5 | 53.2 |
| but-2-enoic acid | 5193.0 | 51.0 |
| 1-hexanol | 5664.5 | 46.5 |
| 4-benzo[1,3]dioxol-5-yl-butan-2-one | 5431.5 | 48.7 |
| phenyl-methanol | 5244.5 | 50.5 |
| furfuryl alcohol | 5585.5 | 47.3 |
| malic acid | 5617.5 | 47.0 |
| 2-ethyl benzenethiol | 4808.5 | 54.6 |
| 2-undecanone | 4581.0 | 56.8 |
| methyl 4-hydroxybenzoate | 4904.5 | 53.7 |
| butyraldehyde | 4725.5 | 55.4 |
| 2-methyl-2-pentenoic acid | 5040.0 | 52.4 |
| 2,5-dimethylphenol | 5208.0 | 50.8 |
| 7-hydroxycitronellal | 6313.0 | 40.4 |
| urea | 5045.5 | 52.4 |
| Compounds tested at 100 µM (Set4, TRPA1 assay) | | |
| H₂O₂ (200 µM | 5881.5 | |
| AITC (50 µM) | 8322.5 | |
| benzyl tiglate | 2385.0 | 59.4 |
| diphenyl disulfide | 1112.0 | 81.1 |
| ethyl acetoacetate | 3314.5 | 43.6 |
| 4-methylthio-2 -butanone | 3322.5 | 43.5 |

### EXAMPLE 3

The compounds listed in TABLE 3 below have been found to be antagonists of the TRPA1 receptor, in that they reduce the level of TRPA1 receptor activation when activated by hydrogen peroxide. Two different levels of antagonist were tested, 100 µM or 400 µM, against 200 µM hydrogen peroxide to determine the level of reduced TRPA1 receptor activation by hydrogen peroxide. Two different level of antagonist were used to demonstrate that certain antagonists require higher levels to provide a significant inhibitory effect.

**TABLE 3**

| Dose of Antagonist | *Cas # | Compound | % Reduction of Hydrogen Peroxide TRPA1 Activation |
|---|---|---|---|
| 100 µM | 8015-91-6 | Cinnamon Bark Oil | 62 |
| 100 µM | 2305-05-7 | γ-Dodecalactone | 51 |
| 100 µM | 121-34-6 | Vanillic Acid | 19 |
| 100 µM | 7011-83-8 | γ-Methyl Decalactone | 48 |
| 400 µM | 8015-91-6 | Cinnamon Bark Oil | 74 |
| 400 µM | 5910-87-2 | trans, trans-2,4-Nonadienal | 67 |
| 400 µM | 6627-88-9 | 4-Allyl-2,6-dimethoxyphenol | 54 |
| 400 µM | 1504-74-1 | o-Methoxycinnamaldehyde | 51 |
| 400 µM | 26643-91-4 | 4-Methyl-2-phenyl-2 Pentenal (mix of cis and trans) | 54 |
| 400 µM | 2785-87-7 | 2-Methoxy-4-propyl-phenol | 43 |
| 400 µM | 606-45-1 | Methyl 2-methoxy-benzoate | 48 |
| 400 µM | 2721-22-4 | δ-Tetradecalactone | |
| 400 µM | 1192-58-1 | 1-Methyl-2-pyrole carboxaldehyde | 42 |
| 400 µM | 710-04-3 | Undecanoic-δ-Lactone | -42 |
| 400 µM | 89-88-3 | (6-Azulenol, 1,2,3,3a,4,5,6,8a-octahydro-4,8-dimethyl-2-(1-methylethylidene)-) Vetiverol | -34 |
| 400 µM | 4166-20-5 | Strawberry Furanone Acetate | 63 |
| 400 µM | 116-02-9 | 3,3,5-Trimethylcyclohexanol | 72 |
| 400 µM | 5422-34-4 | N-(2-Hydroxyethyl)lactamide | 65 |
| 400 µM | 3208-40-0 | 2-(3-Phenylpropyl)tetrahvdrofuran | 74 |
| 400 µM | 6963-56-0 | Anisyl Butyrate | 54 |
| 400 µM | 2046-17-5 | Methyl-4-phenyl butyrate | 60 |
| 400 µM | 40923-64-6 | 3-Heptyldihydro-5-methyl-2(3H)-furanone | 59 |
| 400 µM | 136954-25-1 | 3-acetylsulfanylhexyl acetate | 58 |
| 400 µM | 3720-16-9 | (2-Cyclohexen-1-one, 3-methyl-5-propyl-) Celery Ketone | 56 |
| 400 µM | 85586-67-0 | Isobornyl Isobutyrate | 80 |
| 400 µM | 7549-41-9 | Bornyl Valerate | 52 |
| 400 µM | 4433-36-7 | Citronellyl acetate | 70 |
| 400 µM | 65620-50-0 | (1-Oxaspiro[4.5]decan-6-ol, 2,6,10,10-tetramethyl-, (2S,5S,6S)-6-) Hydroxydihydrotheaspirane | 57 |
| 400 µM | 6728-26-3 | trans-2-Hexenal | 52 |

| | | | |
|---|---|---|---|
| * CAS# refers to the Chemical Abstracts Service system ot classification of chemical entities. | | | |

### EXAMPLE 4

The compounds listed in TABLE 4 below have been found to be antagonists of the TRPV1 receptor, in that they reduce the level of TRPV1 receptor activation when activated by hydrogen peroxide. Different amounts of antagonist (100 µM or 400 µM -depending on the antagonist's ability to reduce hydrogen peroxide's activation of the TRPV1 receptor) were tested against 500 µM hydrogen peroxide to determine the level of reduced TRPV1 receptor activation by hydrogen peroxide.

**TABLE 4**

| Dose of Antagonist | Cas # | Compound | % Reduction of Hydrogen Peroxide TRPV1 Activation |
|---|---|---|---|
| 100 µM | 5655-61-8 | (-)-Bornyl Acetate | 53 |
| 100 µM | 107-75-5 | Hydroxycitronellal | 62 |
| 100 µM | 68133-79-9 | Apritone | 96 |
| 100 µM | 10072-05-6 | Methyl N,N-Dimethylanthranilate | 60 |
| 100 µM | 35243-43-7 | 2-Ethoxy-3 -ethylpyrazine | 58 |
| 100 µM | 4573-50-6 | L-Piperitone | 58 |
| 100 µM | 85586-67-0 | Isobornyl Isobutyrate | 52 |
| 100 µM | 4166-20-5 | 4-Acetoxy-2,5-dimethyl-3(2H)-furanone | 60 |
| 400 µM | 102-69-2 | Tripropylamine | 63 |
| 400 µM | 1128-08-1 | Dihydroj asmone | 98 |
| 400 µM | 1192-58-1 | 1-Methyl-2-pyrole carboxaldehyde | 79 |
| 400 µM | 4864-61-3 | 3-Octyl Acetate | 90 |
| 400 µM | 2445-77-4 | 2-Methylbutyl isovalerate | 79 |
| 400 µM | 51608-18-5 | Jasminone B | 51 |
| 400 µM | 5461-08-5 | Piperonyl Isobutyrate | 63 |
| 400 µM | 23495-12-7 | Phenoxyethyl Propionate | 60 |
| 400 µM | 68527-74-2 | Vanillin Propylene Glycol Acetate | 50 |
| 400 µM | 65737-52-2 | Octenyl Cyclopentanone | 50 |
| 400 µM | 97-87-0 | Butyl Isobutyrate | 58 |
| 400 µM | 8016-23-7 | Guaiacwood Oil | 60 |
| 400 µM | 16409-43-1 | Tetrahydro-4-methyl-2-(2-methyl-1-propenyl)-2H pyran | 53 |

### EXAMPLE 5

The compounds listed in TABLE 5 below have been found to be antagonists of the TRPV1 receptor, in that they reduce the level of TRPV1 receptor activation when activated by L-Menthol. 100 µM of antagonist was tested against 1 mM of L-Menthol to determine the level of reduced TRPV1 receptor activation by L-Menthol. Each antagonist compound was run with 1 mM of L-Menthol as a control and the antagonist activity of each compound was determined by comparing the Ca2+ levels of the control with the antagonist compound being tested.

**TABLE 5**

| Dose of Antagonist | Compounds | Average Ca2+ Counts | % Reduction of L-Menthol TRPV1 Activation |
|---|---|---|---|
| 100 µM | copper(i) iodide | 651.5 | 89.8 |
| 100 µM | butyl 4-hydroxybenzoate | 319.0 | 77.2 |
| 100 µM | diphenyl disulfide | 1630.5 | 64.9 |
| 100 µM | 2,3 -butanedithiol | 586.0 | 64.8 |
| 100 µM | 2,5-dimethyl-1,4-dithiane-2,5-diol | 713.7 | 58.9 |
| 100 µM | cellulose acetate | 2742.5 | 57.1 |
| 100 µM | ethyl 3-phenylglycidate | 1987.0 | 50.8 |
| 100 µM | phenylacetaldehyde | 919.3 | 49.4 |
| 100 µM | 3-phenyl-2-propen-1-yl 3-phenylacrylate | 3377.0 | 47.2 |
| 100 µM | 2,3,5,6-tetramethylpyrazine | 972.0 | 47.0 |
| 100 µM | piperazine | 3405.0 | 46.8 |
| 100 µM | 2-pyrazinylethanethiol | 992.0 | 46.0 |
| 100 µM | 2-methyltetrahydro-3-furanone | 993.7 | 46.0 |
| 100 µM | methyl 3-(methylthio)propionate | 998.7 | 45.7 |
| 100 µM | ethyl 2,4-dioxohexanoate | 1000.3 | 45.7 |
| 100 µM | 3-mercapto-2-butanol | 1008.3 | 45.3 |
| 100 µM | allyl caprylate | 2572.0 | 44.6 |
| 100 µM | 2,5-dimethylpyrazine | 1040.0 | 43.8 |
| 100 µM | succinic acid | 1045.0 | 43.6 |
| 100 µM | hexadecyl lactate | 1047.0 | 43.5 |
| 100 µM | 2-methoxybenzaldehyde | 1050.3 | 43.3 |
| 100 µM | 1,4-dimethoxybenzene | 3633.0 | 43.2 |
| 100 µM | ethyl hexanoate | 3655.5 | 42.9 |
| 100 µM | 3-mercaptobutyl acetate | 1061.3 | 42.8 |
| 100 µM | 2 '-hydroxyacetophenone | 1083.3 | 41.8 |
| 100 µM | ethyl 5-hexenoate | 1084.0 | 41.8 |
| 100 µM | gamma -caprolactone | 1084.3 | 41.8 |
| 100 µM | (+-)-1-phenylethanol | 1087.7 | 41.6 |
| 100 µM | d-(-)-lactic acid | 1093.7 | 41.3 |
| 100 µM | 2-ethyl-3-hydroxy-4h-pyran-4-one | 1095.3 | 41.3 |
| 100 µM | citronellyl formate | 1097.0 | 41.2 |
| 100 µM | ethyl trans-2-hexenoate | 1102.7 | 40.9 |
| 100 µM | propyl pyruvate | 2386.5 | 40.9 |
| 100 µM | o-methylanisole | 1107.3 | 40.7 |
| 100 µM | 1 -isopropyl-4-methylbenzene | 1112.7 | 40.5 |
| 100 µM | trans-2-hexenyl acetate | 1119.0 | 40.2 |
| 100 µM | methyl n-octyl sulfide | 1120.3 | 40.1 |
| 100 µM | propionic acid trans-2-hexen-1-yl ester | 1128.3 | 39.7 |
| 100 µM | choline bitartrate | 1141.3 | 39.1 |
| 100 µM | 2-methyl-3-furanthiol | 2835.5 | 38.9 |
| 100 µM | crotonic acid cis-3-hexen-1-yl ester | 1150.0 | 38.7 |
| 100 µM | manganese chloride | 3922.0 | 38.7 |
| 100 µM | 4'-methoxyacetophenone | 1162.0 | 38.2 |
| 100 µM | empg | 1164.0 | 38.1 |
| 100 µM | zinc gluconate | 3963.5 | 38.1 |
| 100 µM | trans-2-hexen-1-al diethyl acetal | 1169.0 | 37.9 |
| 100 µM | 1-decanol | 1175.7 | 37.5 |
| 100 µM | methyl 2-(acetylamino)benzoate | 1178.3 | 37.4 |
| 100 µM | 2-isobutyl-3 -methoxypyrazine | 4020.0 | 37.2 |
| 100 µM | methyl laurate | 1199.7 | 36.4 |
| 100 µM | 4-ethoxybenzaldehyde | 1200.0 | 36.4 |
| 100 µM | 2-benzoylamino-benzoic acid | 1207.7 | 36.1 |
| 100 µM | phenylpropanol, phenylpropanol (1-phenyl 1-propanol) | 1218.3 | 35.6 |
| 100 µM | 4-methyl-5-vinylthiazole | 1227.7 | 35.1 |
| 100 µM | butyl 2-methylbutyrate | 1233.7 | 34.9 |
| 100 µM | edta | 1236.0 | 34.8 |
| 100 µM | ocimene quintoxide | 1236.3 | 34.7 |
| 100 µM | ferulic acid | 1242.0 | 34.5 |
| 100 µM | 2-methyl-1,3-dithiolane | 2650.5 | 34.3 |
| 100 µM | 2-acetyl-1-methylpyrrole | 1254.0 | 33.9 |
| 100 µM | 2,4-dimethylanisole | 1255.3 | 33.9 |
| 100 µM | tetrahydrofurfuryl acetate | 1269.3 | 33.2 |
| 100 µM | 4-methyl-1-phenyl-2-pentanone | 2707.0 | 32.9 |
| 100 µM | ethyl propionate | 2712.0 | 32.8 |
| 100 µM | 2-undecanone | 1282.3 | 32.6 |
| 100 µM | 4-methyl-2,6-dimethoxyphenol | 1292.3 | 32.1 |
| 100 µM | diallyl sulfide | 1293.3 | 32.1 |
| 100 µM | beta-caryophyllene | 2744.5 | 32.0 |
| 100 µM | iron naphthenate | 2748.5 | 31.9 |
| 100 µM | octanal | 2759.0 | 31.6 |
| 100 µM | 2-chloroacetophenone | 1313.0 | 31.2 |
| 100 µM | beta-resorcylic acid | 1317.3 | 31.0 |
| 100 µM | ethyl 3-(2-furyl)propionate | 4446.5 | 30.5 |
| 100 µM | (1r)-(+)-camphor | 2807.0 | 30.5 |
| 100 µM | thymol | 1331.3 | 30.3 |
| 100 µM | dextrin from potato starch | 1336.0 | 30.1 |
| 100 µM | 2,3,6-trimethylphenol | 1339.3 | 30.0 |
| 100 µM | isopropyl tiglate | 4497.5 | 29.7 |
| 100 µM | 2-sec.-butylcyclohexanone | 1352.3 | 29.4 |
| 100 µM | 2,6-dimethoxyphenol | 1365.0 | 28.8 |
| 100 µM | phloretin | 4616.5 | 27.9 |
| 100 µM | benzyl formate | 2923.5 | 27.6 |
| 100 µM | dibutyl sebacate | 1398.0 | 27.3 |
| 100 µM | 4-methoxybenzyl formate | 1399.0 | 27.2 |
| 100 µM | (+)-sodium 1-ascorbate | 2961.0 | 26.6 |
| 100 µM | hexanedioic acid, dipropyl ester | 2964.0 | 26.6 |
| 100 µM | (r)-(+)-2-phenyl-1-propanol | 1414.0 | 26.5 |
| 100 µM | 5 -methyl-2,3 -hexanedione | 2968.5 | 26.4 |
| 100 µM | 1-stearoyl-rac-glycerol | 4708.0 | 26.4 |
| 100 µM | 4-methoxyphenylacetone | 1421.7 | 26.2 |
| 100 µM | cis-3-octen-1-ol | 1424.7 | 26.0 |
| 100 µM | cis-3-hexenyl butyrate | 2987.5 | 26.0 |
| 100 µM | 3 -(methylthio)propyl isothiocyanate | 1426.7 | 25.9 |
| 100 µM | isobutyl hexanoate | 3443.5 | 25.8 |
| 100 µM | ethyl oleate | 1431.3 | 25.7 |
| 100 µM | 2-ethyl-3 -methoxypyrazine | 1432.7 | 25.7 |
| 100 µM | 2-methoxybenzoic acid | 1441.7 | 25.2 |
| 100 µM | 1-octanol | 1442.3 | 25.2 |
| 100 µM | 2,5-dimethylfuran-3-thiol | 1443.0 | 25.2 |
| 100 µM | 6,6-dimethylbicyclo[3.1.1]hept-2-ene-2-methyl acetate | 1448.3 | 24.9 |
| 100 µM | isopentyl formate | 1451.7 | 24.8 |
| 100 µM | ethyl trans-4-decenoate | 1454.3 | 24.6 |
| 100 µM | allyl cyclohexanepropionate | 4895.5 | 23.5 |
| 100 µM | 2-(1-propoxyethoxy)ethylbenzene | 1480.7 | 23.4 |
| 100 µM | methyl nicotinate | 1483.7 | 23.3 |
| 100 µM | 2-acetylthiazole | 3569.5 | 23.1 |
| 100 µM | (+-)-beta;-citronellol | 1501.3 | 22.5 |
| 100 µM | pyruvic aldehyde | 1504.0 | 22.3 |
| 100 µM | quinine hydrochloride dihydrate | 1505.3 | 22.3 |
| 100 µM | sec-butyl disulfide | 3652.0 | 21.3 |
| 100 µM | methyl 2-methylpentanoate | 1529.3 | 21.2 |
| 100 µM | methyl trans-2-octenoate | 1538.7 | 20.7 |
| 100 µM | 3,4-dihydroxybenzoic acid | 1539.7 | 20.7 |
| 100 µM | ethyl 2-benzylacetoacetate | 1548.3 | 20.3 |
| 100 µM | 3-methylthio-2-butanone | 1554.0 | 20.0 |

As demonstrated in EXAMPLES 6 and 7 described below, the negative attributes of menthol and hydrogen peroxide can be reduced using TRPA1 and TRPV1 antagonists. These reductions translate into a user noticeable signal from the personal care product.

For EXAMPLES 6 and 7 sensory evaluation studies of menthol and hydrogen peroxide activity were conducted using a methodology patterned after the techniques described in M. C. Meilgaard, et al., Sensory Evaluation Techniques, 4th Ed. (2007). In one study, a panel of 8-15 trained sensory experts evaluated the menthol or peroxide sensations experienced after brushing with a dentifrice containing the menthol or hydrogen peroxide and the respective TRPA1 or TRPV1 antagonists. Panelists brushed teeth with 1.5 grams of a dentifrice (containing coolant) or control (no coolant) and then expectorated. After brush expectoration, panelists evaluated the burn intensity, assigning a number between 0 (no burning) to 60 (intense cooling). After rinse expectoration, panelists evaluated burning intensity according to the same 0 to 60 scale. Evaluations were conducted at 5, 15, 30, 45, 60 minute, etc. time points. At each evaluation, panelists were instructed to breathe in through pursed lips and evaluate overall burning sensation. In this test, a numerical score difference from the control of 7.5 indicates user significant differences or definite reduction in burning at the specified time point. Measures between 4.0 to 7.5 indicate a noticeable trend in the specified parameter, which is not statistically significant, but noticeable.

### EXAMPLE 6

For EXAMPLE 6 (TABLES 8 and 9), a panel of 14 trained sensory experts evaluated the sensory profile experienced after brushing with the dentifrice sample formulations shown in TABLES 6 and 7 containing high levels of menthol followed by rinsing with water. Panelists brushed teeth with 1.5 grams of a test dentifrice (containing high menthol levels) or control (no menthol) for 36 seconds and then expectorated. The dentifrice sample formulations (control and high L-Menthol) are shown below in TABLE 6 (with peppermint flavor) and TABLE 7 (with spearmint flavor). The dentifrices were made using conventional methods and are shown below with component amounts in weight % of total composition.

**TABLE 6**

| Ingredient | A (Control) | B | C | D | E | F | G | H | I |
|---|---|---|---|---|---|---|---|---|---|
| FD&C Blue #1 Color Solution | 0.045% | 0.045% | 0.045% | 0.045% | 0.045% | 0.045% | 0.045% | 0.045% | 0.045% |
| Sodium Fluoride | 0.243% | 0.243% | 0.243% | 0.243% | 0.243% | 0.243% | 0.243% | 0.243% | 0.243% |
| CARBOMER 956 | 0.300% | 0.300% | 0.300% | 0.300% | 0.300% | 0.300% | 0.300% | 0.300% | 0.300% |
| Sodium Saccharin | 0.300% | 0.300% | 0.300% | 0.300% | 0.300% | 0.300% | 0.300% | 0.300% | 0.300% |
| Sodium Phosphate, Monobasic, Monohydrate | 0.419% | 0.419% | 0.419% | 0.419% | 0.419% | 0.419% | 0.419% | 0.419% | 0.419% |
| Titanium Dioxide | 0.525% | 0.525% | 0.525% | 0.525% | 0.525% | 0.525% | 0.525% | 0.525% | 0.525% |
| Carboxymethycell ulose Sodium | 0.800% | 0.800% | 0.800% | 0.800% | 0.800% | 0.800% | 0.800% | 0.800% | 0.800% |
| Peppermint Flavor | 1.000% | 1.000% | 1.000% | 1.000% | 1.000% | 1.000% | 1.000% | 1.000% | 1.000% |
| Spearmint Flavor | 0% | 0% | 0% | 0% | 0% | 0% | 0% | 0% | 0% |
| Added L-Menthol | 0% | 0.25% | 0.5% | 0.75% | 1.0% | 1.25% | 1.5% | 1.75% | 2.0% |
| Tribasic Sodium Phosphate Dodecahydrate | 1.100% | 1.100% | 1.100% | 1.100% | 1.100% | 1.100% | 1.100% | 1.100% | 1.100% |
| Sodium Lauryl Sulfate 28% Solution | 4.000% | 4.000% | 4.000% | 4.000% | 4.000% | 4.000% | 4.000% | 4.000% | 4.000% |
| Silica, Dental Type, NF (Zeodent 119) | 15.000% | 15.000 % | 15.000 % | 15.000 % | 15.000 % | 15.000 % | 15.000 % | 15.000 % | 15.000 % |
| SORBITOL SOLUTION LRS USP | 54.673% | 54.673 % | 54.673 % | 54.673 % | 54.673 % | 54.673 % | 54.673 % | 54.673 % | 54.673 % |
| Water Purified, USP, PhEur, JP, JSCI | QS* | QS* | QS* | QS* | QS* | QS* | QS* | QS* | QS* |

| | | | | | | | | | |
|---|---|---|---|---|---|---|---|---|---|
| *QS refers to the term *quantum sufficit,* meaning as much as suffices, where the remainder of the formula hole is filled with this substance | | | | | | | | | |

**TABLE 7**

| Ingredient | J (Contro l) | K | L | M | N | O | P | Q | R |
|---|---|---|---|---|---|---|---|---|---|
| FD&C Blue #1 Color Solution | 0.045% | 0.045% | 0.045% | 0.045% | 0.045% | 0.045% | 0.045% | 0.045 % | 0.045 % |
| Sodium Fluoride | 0.243% | 0.243% | 0.243% | 0.243% | 0.243% | 0.243% | 0.243% | 0.243 % | 0.243 % |
| CARBOMER 956 | 0.300% | 0.300% | 0.300% | 0.300% | 0.300% | 0.300% | 0.300% | 0.300 % | 0.300 % |
| Sodium Saccharin | 0.300% | 0.300% | 0.300% | 0.300% | 0.300% | 0.300% | 0.300% | 0.300 % | 0.300 % |
| Sodium Phosphate, Monobasic, Monohydrate | 0.419% | 0.419% | 0.419% | 0.419% | 0.419% | 0.419% | 0.419% | 0.419 % | 0.419 % |
| Titanium Dioxide | 0.525% | 0.525% | 0.525% | 0.525% | 0.525% | 0.525% | 0.525% | 0.525 % | 0.525 % |
| Carboxymethycellul ose Sodium | 0.800% | 0.800% | 0.800% | 0.800% | 0.800% | 0.800% | 0.800% | 0.800 % | 0.800 % |
| Peppermint Flavor | 0% | 0% | 0% | 0% | 0% | 0% | 0% | 0% | 0% |
| Spearmint Flavor | 1.000% | 1.000% | 1.000% | 1.000% | 1.000% | 1.000% | 1.000% | 1.000 % | 1.000 % |
| Added L-Menthol | 0% | 0.25% | 0.5% | 0.75% | 1.0% | 1.25% | 1.5% | 1.75% | 2.0% |
| Tribasic Sodium Phosphate Dodecahydrate | 1.100% | 1.100% | 1.100% | 1.100% | 1.100% | 1.100% | 1.100% | 1.100 % | 1.100 % |
| Sodium Lauryl Sulfate 28% Solution | 4.000% | 4.000% | 4.000% | 4.000% | 4.000% | 4.000% | 4.000% | 4.000 % | 4.000 % |
| Silica, Dental Type, NF (Zeodent 119) | 15.000 % | 15.000 % | 15.000 % | 15.000 % | 15.000 % | 15.000 % | 15.000 % | 15.000 % | 15.000 % |
| SORBITOL SOLUTION LRS USP | 54.673 % | 54.673 % | 54.673 % | 54.673 % | 54.673 % | 54.673 % | 54.673 % | 54.673 % | 54.673 % |
| Water Purified, USP, PhEur, JP, JSCI | QS* | QS* | QS* | QS* | QS* | QS* | QS* | QS* | QS* |

| | | | | | | | | | |
|---|---|---|---|---|---|---|---|---|---|
| *QS refers to the term *quantum sufficit,* meaning as much as suffices, where the remainder of the formula hole is filled with this substance | | | | | | | | | |

TABLES 6 and 7 contain the formulations for toothpastes with added menthol going from 0% (control toothpaste for each flavor type) to 2.0% additional menthol for a 1% peppermint toothpaste (Samples A-I-TABLE 6) and for a 1% spearmint toothpaste (Samples J-R -TABLE 7).

Trained panelists separately brushed their teeth with each of the toothpastes (A-R) and rated them separately according to perceived burn. After expectoration, the panelists rinsed their mouth with 15 mls of tap water at room temperature (average temperature of 20° c). The 14 panelists began the rating the burn sensation measurement in-mouth during brushing and after expectoration &water rinse over the course of 20 minutes. They assessed the burn sensation in their mouth and on their lips at 0 minutes after expectoration, 5 minutes, 10 minutes, 15 minutes, and 20 minutes after expectoration. They assigned a burn sensation of 0 (no sensation) to 60 (highest intensity sensation). At each evaluation, panelists were instructed to breathe in through pursed lips and evaluate overall sensation. In this test, a numerical score of 7.5 indicates a significant user noticeable sensation. Differences less than 7.5, but greater than 5.0 indicate the trending of the data on a specific attribute. Results are shown in TABLE 8 for peppermint toothpaste (Samples A-I - TABLE 6) and 9 for spearmint toothpaste (Samples J-R -TABLE 7) below.

**TABLE 8**

| Sample | Burn sensation rating for peppermint | | | | | | | | |
|---|---|---|---|---|---|---|---|---|---|
| | 6A | 6B | 6C | 6D | 6E | 6F | 6G | 6H | 6I |
| In-mouth | 6.3 | 14.3 | 17.6 | 15.9 | 21.8 | 22.6 | 21.5 | 23.9 | 29.9 |
| After Expectoration | 13.7 | 17.6 | 24.2 | 25.7 | 34.3 | 33.9 | 37.5 | 33.7 | 42.0 |
| 0 min after rinse | 10.3 | 13.9 | 20.5 | 20.7 | 28.4 | 31.9 | 33.3 | 32.2 | 38.0 |
| 5 min after rinse | 3.5 | 2.3 | 9.5 | 7.2 | 10.2 | 11.2 | 15.7 | 11.2 | 17.5 |
| 10 min after rinse | 1.0 | 0.0 | 2.1 | 1.3 | 3.6 | 4.5 | 7.2 | 5.3 | 7.0 |
| 15 min after rinse | 0.8 | 0.0 | 0.7 | 0.6 | 2.3 | 2.1 | 3.3 | 1.5 | 3.8 |
| 20 min after rinse | 0.5 | 0.0 | 0.4 | 0.4 | 0.6 | 1.3 | 1.5 | 0.8 | 1.5 |

**TABLE 9**

| Sample # | Burn sensation rating for spearmint | | | | | | | | |
|---|---|---|---|---|---|---|---|---|---|
| | 7J | 7K | 7L | 7M | 7N | 7O | 7P | 7Q | 7R |
| In-mouth | 31.1 | 33.0 | 37.1 | 36.9 | 40.9 | 40.0 | 42.5 | 40.4 | 44.4 |
| 0 min after rinse | 33.3 | 33.6 | 38.0 | 38.2 | 41.9 | 40.5 | 44.1 | 42.3 | 46.1 |
| 5 min after rinse | 21.3 | 22.1 | 31.0 | 28.3 | 32.0 | 31.8 | 35.1 | 35.1 | 38.1 |
| 10 min after rinse | 7.4 | 13.3 | 21.0 | 12.8 | 21.6 | 20.4 | 25.9 | 26.9 | 29.9 |
| 15 min after rinse | 2.8 | 6.0 | 10.0 | 6.3 | 14.5 | 10.9 | 12.1 | 14.7 | 19.5 |
| 20 min after rinse | 1.8 | 2.9 | 7.1 | 3.3 | 5.8 | 4.5 | 7.2 | 7.5 | 8.8 |

As shown in TABLE 8, the burn sensation increases significantly for dentifrices which contain >0.25% added menthol (Samples B-I). The burn sensation was significant for the 0.5% added menthol and higher formulations (Samples C-I) for peppermint when compared to the control (Sample A)which contained the flavor without the additional menthol. The burn sensation for the peppermint formulations with >0.25% (Samples B-I) added menthol was significantly high for 5-10 minutes after the water rinse.

As shown in TABLES 8 and 9 the burn sensation for Samples having spearmint (Samples J-R) was higher than that of Samples having peppermint (Samples A-I). This is exemplified in the Samples having no menthol, Sample A for peppermint and Sample J for spearmint. When >0.25% menthol was added (Samples K-R), the burn sensation was significantly higher. The burn sensation was significantly higher for the 0.5% added menthol and higher formulations (Samples L-R) for spearmint when compared to the control formulation (Sample J) which contained the flavor without the additional menthol. The burn sensation for the spearmint formulations was significantly high for 5-20 minutes after the water rinse.

TABLES 8 and 9 demonstrate the need to add burn sensation antagonists when adding high levels of menthol to the formulations.

For TABLE 10 all the Samples had the same formulation as Sample E from TABLE 6, except for the Control which lacked menthol. After brushing or mouthwash expectoration, panelists evaluated the burning sensation, assigning a number between 0 (no sensation) to 60 (highest intensity sensation). Evaluations were conducted at 5, 10, 20 and 30 minute time points, following expectoration. At each evaluation, panelists were instructed to breathe in through pursed lips and evaluate overall sensation. In this test, a numerical score of 7.5 indicates a significant user noticeable sensation. Differences less than 7.5, but greater than 5.0 indicate the trending of the data on a specific attribute. Results are shown in TABLE 10 below.

**TABLE 10**

| | Burning Sensation Rating | | | | | |
|---|---|---|---|---|---|---|
| Sample | After Expectoration | After Rinse | 5 Min After Rinse | 10 Min After Rinse | 20 Min After Rinse | 30 Min After Rinse |
| Control (no menthol) | 14.3 | 10.6 | 1.2 | 0.0 | 0.0 | 0.0 |
| Control + 1% Menthol | 25.6 | 20.8 | 12.2 | 3.9 | 0.8 | 0.1 |
| 200 ppm Apritone | 27.4 | 24.0 | 13.3 | 5.3 | 0.0 | 0.0 |
| 6 ppm Dihydrojasmone | 24.1 | 21.3 | 11.3 | 2.5 | 0.0 | 0.0 |
| 100 ppm Isobornyl Isobutyrate | 25.4 | 22.8 | 12.8 | 5.6 | 0.6 | 0.0 |
| 100 ppm Isobornyl Isobutyrate +200 ppm Apritone | 23.9 | 20.6 | 6.6 | 1.9 | 0.0 | 0.1 |
| 100 ppm Isobornyl Isobutyrate+6 ppm Dihydrojasmone | 25.6 | 22.4 | 10.0 | 0.9 | 0.0 | 0.0 |
| 100 ppm α-heptyl-γ-valerolactone | 25.4 | 21.5 | 13.8 | 1.3 | 0.0 | 0.0 |
| 100 ppm α-heptyl-γ-valerolactone + 200 ppm Apritone | 21.8 | 19.1 | 9.3 | 2.8 | 0.0 | 0.0 |
| 100 ppm α-heptyl-γ-valerolactone + 6 ppm Dihydrojasmone | 26.1 | 21.7 | 10.2 | 2.5 | 0.0 | 0.0 |

The data in TABLE 10 was from panel testing of a dentifrice (Sample E from TABLE 6) containing TRPV1 and/or TRPA1 antagonists. As shown in TABLE 10, overall, the combination of apritone plus isobornyl isobutyrate showed significant reductions in perceived burning sensation from 1% menthol, as compared to the control + 1% menthol, at 5 min after brushing (6.6 for isobornyl isobutyrate + apritone compared to 12.2 for control + menthol) and 10 min after brushing (1.9 for isobornyl isobutyrate + apritone compared to 3.9 for control + menthol) with the dentifrice and rinsing with 15 mls room temperature water. This combination with menthol showed the trend of reducing the burning sensation.

### EXAMPLE 7

For EXAMPLE 7, a panel of 10 trained sensory experts evaluated the sensory profile experienced after rinsing with 30 grams of a test mouthwash (containing hydrogen peroxide and TRPA1 or TRPV1 antagonist) or control (containing hydrogen peroxide and no TRPA1 or TRPV1 antagonist) for 30 seconds and then expectorated. After expectoration, panelists evaluated the sensorial profile (cooling, warming, burning, numbing, and astringency), assigning a number between 0 (no sensation) to 60 (highest intensity sensation). Evaluations were conducted at 2.5, 5, 10, 20, and 30 minute time points, following expectoration at each evaluation, panelists were instructed to breathe in through pursed lips and evaluate overall burning or numbing sensation. The hydrogen peroxide containing mouthwash formulations are shown below in TABLE 11. The mouthwash samples tested comprised a control having the components listed in TABLE 11 by weight % of the composition or the control formulation plus TRPA1 or TRPV1 antagonist, as shown in TABLE 12. The mouthwashes were made using conventional methods and are shown below with amounts in weight % of total composition. Results are shown in TABLE 12 below.

**TABLE 11**

| Ingredient | Control |
|---|---|
| 35% H₂O₂ solution | 4.286 |
| Sensate** | 0.0-0.1 |
| Flavor | 0.01-0.3 |
| Poloxamer 407 | 0.05-0.2 |
| Glycerin | 11-25 |
| Propylene Glycol | 0.01-3.00 |
| Sodium Saccharin | 0.01-0.1 |
| Cetyl Pyridinium Chloride | 0.1025% |
| Phosphoric Acid | 0.0-0.02 |
| Water, Purified, USP | QS* |

| | |
|---|---|
| *QS refers to the term *quantum sufficit,* meaning as much as suffices, where the remainder of the formula hole is filled with this substance **Sensates can be chosen from the non-limiting examples of cooling agents, warming agents, tingle agents, numbing agents, or combinations thereof. | |

**TABLE 12**

| | Burning Sensation | | | | | | |
|---|---|---|---|---|---|---|---|
| | Initial in Mouth | After Expectora tion | 2 Min 30 SEC After Expectoration | 5 Min After Expectoratio n | 10 Min After Expectoration | 20 Min After Expectoration | 30 Min After Expectoration |
| Control Mouthwash | 18.2 | 24.9 | 17.6 | 11.7 | 3.6 | 1.7 | 0.0 |
| 200 ppm Apritone | 12.6 | 21.9 | 20.8 | 16.0 | 8.6 | 3.3 | 1.3 |
| 100 ppm Isobornyl Isobutyrate | 12.7 | 20.9 | 17.6 | 11.4 | 5.1 | 0.0 | 0.0 |
| 200 ppm Apritone + 100 ppmIsobornyl Isobutyrate | 10.7 | 17.4 | 14.1 | 5.3 | 2.8 | 0.3 | 0.0 |
| 200 ppm Apritone + 6 ppm Dihydrojasmone | 12.6 | 19.1 | 14.1 | 8.9 | 1.7 | 0.0 | 0.0 |
| 6 ppm Dihydrojasmone | 12.5 | 16.9 | 13.1 | 8.9 | 2.8 | 0.8 | 0.6 |
| 100 ppm | 12.9 | 22.4 | 20.6 | 15.4 | 5.6 | 0.0 | 0.0 |

| | Numbing Sensation | | | | | | |
|---|---|---|---|---|---|---|---|
| | Initial in Mouth | After Expectora tion | 2 Min 30 SEC After Expectoration | 5 Min After Expectoratio n | 10 Min After Expectoration | 20 Min After Expectoration | 30 Min After Expectoration |
| Control Mouthwash | NR | 11.8 | 10.9 | 9.8 | 5.6 | 3.1 | 0.0 |
| 200 ppm Apritone | NR | 11.6 | 12.4 | 10.1 | 6.0 | 4.2 | 0.8 |
| 100 ppm Isobornyl Isobutyrate | NR | 8.4 | 7.9 | 5.8 | 2.5 | 0.0 | 0.0 |
| 200 ppm Apritone + 100 ppm Isobornyl Isobutyrate | NR | 7.3 | 6.1 | 2.5 | 0.0 | 0.0 | 0.0 |
| 200 ppm Apritone + 6 ppm Dihydrojasmone | NR | 8.4 | 7.5 | 5.7 | 0.6 | 0.0 | 0.0 |
| 6 ppm Dihydrojasmone | NR | 6.9 | 6.9 | 6.3 | 0.8 | 0.0 | 0.0 |
| 100 ppm | NR | 7.9 | 8.5 | 6.9 | 3.3 | 0.6 | 0.0 |

As shown in TABLE 12, the mouthwash compositions provide a pleasant high-impact minty taste during use and noticeable long-lasting fresh breath with a reduction in burning and numbing sensations from hydrogen peroxide in mouthwash.

### EXAMPLE 8

For EXAMPLE 8, 50 panelists evaluated the sensory profile experienced after brushing with a zinc based dentifrice containing 0.4% menthol for 2 minutes, expectorating the dentifrice, then rinsing with 15ml tap water at room temperature and then expectorating. The high menthol containing dentifrice formulations (control and with antagonist) are shown below in TABLE 13. The dentifrices were made using conventional methods and are shown below with amounts in weight % of total composition. The commercial product was purchased at a local store and evaluated as shown.

**TABLE 13**

| | Samples | | |
|---|---|---|---|
| Ingredient | A | B | C |
| Mica, Titanium Dioxide coated | 0.4% | 0.4% | 0.4% |
| Sodium Fluoride | 0.243% | 0.243% | 0.243% |
| Polyethylene Specks, Blue | 0.35% | 0.35% | 0.35% |
| Carrageenan | 0.7% | 0.7% | 0.7% |
| Sodium Saccharin | 0.300% | 0.300% | 0.300% |
| Titanium Dioxide | 0.525% | 0.525% | 0.525% |
| Carboxymethycellulose Sodium | 1.3% | 1.3% | 1.3% |
| Hydroxyethylcellulose | 0.3% | 0.3% | 0.3% |
| Peppermint Flavor | 1.000% | 1.000% | 1.000% |
| Added Menthol | 0% | 0.25% | 0.25% |
| Sodium Lauryl Sulfate 28% Solution | 1.0% | 1.0% | 1.0% |
| Silica, Dental Type, NF (Zeodent 119) | 17% | 17% | 17% |
| Sorbitol Solution LRS USP | 40.5% | 40.5% | 40.5% |
| Zinc Citrate Dihydrate | 0.788% | 0.788% | 0.788% |
| Stannous Chloride Dihydrate | 0.209% | 0.209% | 0.209% |
| Apritone | 0% | 0.03% | 0% |
| Isobornyl Isobutyrate | 0% | 0.005% | 0% |
| G180 coolant | 0.025% | 0.010% | 0.010% |
| Vanillyl Butyl Ether | 0% | 0% | 0% |
| Zingerone | 0% | 0% | 0% |
| Frescolat MGA coolant | 0.0225% | 0.010% | 0.010% |
| WS5 coolant | 0.007% | 0.010% | 0% |
| Sucralose | 0.2% | 0.2% | 0.2% |
| Water Purified, USP | QS* | QS* | QS* |

| | | | |
|---|---|---|---|
| *QS refers to the term *quantum sufficit,* meaning as much as suffices, where the remainder of the formula hole is filled with this substance | | | |

**TABLE 14**

| | Samples | | |
|---|---|---|---|
| n = 50 Avg. Scores Significance level=90% | A | B | C |
| Overall Flavor Rating | 62 | 67 | 61 |
| Pleasant after taste in mouth | 64 | 68 | 62 |
| Overall Acceptance Rating | 62 | 64 | 60 |
| Overall Acceptance Rating at 30 minutes post brushing | 59 | 62 | 60 |

TABLE 14 shows the panelists' response to the formulations in TABLE 13. Sample B had the higher menthol plus the apritone plus isobornyl isobutyrate and it scored the highest overall flavor rating, and also had the highest pleasant aftertaste in mouth. After 30 minutes, Sample B had the highest overall acceptance rating.

### EXAMPLE 9

The compounds listed in TABLE 15 below have been found to be antagonists of the TRPA1 receptor, in that they reduce the level of TRPA1 receptor activation when activated by hydrogen peroxide or L-Menthol. 100 µM of antagonist was tested against 1 mM of L-Menthol or 500 µM hydrogen peroxide to determine if they impeded TRPA1 receptor activation by hydrogen peroxide, L-Menthol, or both. TABLE 15 below illustrates the antagonists specific to either L-Menthol or hydrogen peroxide and those that antagonize both L-Menthol and hydrogen peroxide. Intracellular Ca⁺⁺ levels were measured using cell based assays as described for EXAMPLES 1, 2, and 3.

**TABLE 15**

| **BLOCK ONLY H2O2/TRPA1** | Dose |
|---|---|
| | |
| 2-Hexyl-4-methyl-1,3-dioxolane | 100 µM |
| Fenchone | 100 µM |
| 2-methyl-3-ethoxypyrazine | 100 µM |
| Isopropyl Hexanoate | 100 µM |
| 2-Methyl-1-Butanethiol | 100 µM |
| Desoxycholic acid | 100 µM |
| n-Butyl Alcohol | 100 µM |
| Sodium Erythorbate | 100 µM |
| 1-Undecanol | 100 µM |
| 6-Undecanone | 100 µM |
| 3-acetylpyridine, 98% | 100 µM |
| Amyl Alcohol | 100 µM |
| dl-Verbenone | 100 µM |
| Methyl Linoleate | 100 µM |
| Acetic acid isopropenyl ester | 100 µM |
| 3-Mercapto-2-Pentanone | 100 µM |
| 3-Hydroxybenzoic Acid | 100 µM |
| Phenylethyl Isovalerate | 100 µM |
| 2-Acetyl-5-methylfuran | 100 µM |
| 2-Nonanol | 100 µM |
| Isoamyl Pyruvate | 100 µM |
| Ethyl Methyl Beta-Phenylethyl Carbinol | 100 µM |
| Ferric Chloride | 100 µM |
| Alpha, Alpha-dimethyl hydrocinnamyl acetate | 100 µM |
| 1-Octen-3-yl acetate | 100 µM |
| gamma-Terpinene | 100 µM |
| 2-Undecanol | 100 µM |
| 3-Methyl-1,2-cyclopentanedione | 100 µM |
| P-Cymen-8-ol | 100 µM |
| Quinoline | 100 µM |
| 2-Methyl butyl 2-methylbutyrate | 100 µM |
| 4'-Methylacetophenone | 100 µM |
| 3-Hexanone | 100 µM |
| Isobutyl isobutyrate | 100 µM |
| 2-Phenyl-2-Butenal | 100 µM |
| Alpha-p-dimethylstyrene | 100 µM |
| Isopentyl Alcohol | 100 µM |
| Methylbenzoate | 100 µM |
| 2-Methyl-3-heptanone | 100 µM |
| 3,7-Dimethyl-1-octanol | 100 µM |
| 2,4,6-Trithiaheptane | 100 µM |
| 5-Methylhexanoic acid | 100 µM |
| Acetoin | 100 µM |
| 2-Propionylthiazole | 100 µM |
| Benzothiazole | 100 µM |
| Butyl lactate | 100 µM |
| 2-Ethyl-1-hexanol | 100 µM |
| But-2-enoic acid | 100 µM |
| 1-hexanol | 100 µM |
| 4-Benzo[1,3]dioxol-5-yl-butan-2-one | 100 µM |
| Phenyl-methanol | 100 µM |
| malic acid | 100 µM |
| Methyl 4-hydroxybenzoate | 100 µM |
| Butyraldehyde | 100 µM |
| 2-Methyl-2-pentenoic acid | 100 µM |
| 2,5-Dimethylphenol | 100 µM |
| 7-Hydroxycitronellal | 100 µM |
| Urea | 100 µm |
| Benzyl Tiglate | 100 µM |
| γ-Dodecalactone | 100 µM |
| Vanillic Acid | 100 µM |
| γ-Methyl Decalactone | 100 µM |
| 2-Methoxy-4-propyl-phenol | 400 µM |
| Methyl 2-methoxy-benzoate | 400 µM |
| 1-Methyl-2-pyrole carboxaldehyde | 400 µM |
| Strawberry Furanone Acetate | 400 µM |
| 3,3,5 -Trimethylcyclohexanol | 400 µM |
| N-(2-Hydroxyethyl)lactamide | 400 µM |
| 2-(3-Phenylpropyl) tetrahydrofuran | 400 µM |
| Methyl-4-phenyl butyrate | 400 µM |
| 3-Heptyldihydro-5-methyl-2(3H)-furanone | 400 µM |
| 3-acetylsulfanylhexyl acetate | 400 µM |
| Isobornyl Isobutyrate | 400 µM |
| Bornyl Valerate | 400 µM |
| Citronellyl acetate | 400 µM |
| Trans-2-hexenal | 400 µM |
| (2-Cyclohexen-1-one, 3-methyl-5-propyl-) Celery Ketone | 400 µM |
| (1-Oxaspiro[4.5]decan-6-ol, 2,6,10,10-tetramethyl-, (2S,5S,6S)-6-)Hydroxydihydrotheaspirane | 400 µM |
| | |

| **BLOCK ONLY MENTHOL/TRPA1** | Dose |
|---|---|
| | |
| Ethyl Hexanoate | 100 µM |
| 1,4-Dimethoxybenzene | 100 µM |
| Cellulose acetate | 100 µM |
| 3-phenyl-2-propen-1-yl 3-phenylacrylate | 100 µM |
| Isopropyl Tiglate | 100 µM |
| Ethyl 3-(2-Furyl)Propionate | 100 µM |
| Iron Naphthenate | 100 µM |
| Beta-Caryophyllene | 100 µM |
| (+)-Sodium L-ascorbate | 100 µM |
| 5-Methyl-2,3-Hexanedione | 100 µM |
| Ethyl propionate | 100 µM |
| Ethyl 3-phenylglycidate | 100 µM |
| Propyl Pyruvate | 100 µM |
| Octanal | 100 µM |
| 4-Methyl-1-Phenyl-2-Pentanone | 100 µM |
| Benzyl formate | 100 µM |
| cis-3-hexenyl butyrate | 100 µM |
| (1R)-(+)-Camphor | 100 µM |
| Hexanedioic acid, Dipropyl ester | 100 µM |
| trans-2 -Hexenyl acetate | 100 µM |
| Ethyl 2,4-Dioxohexanoate | 100 µM |
| o-Methylanisole | 100 µM |
| Methyl N-Octyl Sulfide | 100 µM |
| (+-)-beta;-Citronellol | 100 µM |
| 3-Mercaptobutyl acetate | 100 µM |
| 2,5-Dimethylpyrazine | 100 µM |
| 2-Benzoylamino-benzoic acid | 100 µM |
| Angelic Acid Isobutyl Ester | 100 µM |
| 2,3,6-Trimethylphenol | 100 µM |
| Ethyl 2-benzylacetoacetate | 100 µM |
| 1-isopropyl-4-methylbenzene | 100 µM |
| 2,3,5,6-Tetramethylpyrazine | 100 µM |
| trans-Cinnamaldehyde | 100 µM |
| Diallyl sulfide | 100 µM |
| Pyruvic Aldehyde | 100 µM |
| 1-octanol | 100 µM |
| Choline bitartrate | 100 µM |
| 3, 4-Dihydroxybenzoic acid | 100 µM |
| 4-Methyl-2,6-Dimethoxyphenol | 100 µM |
| Ethyl trans-2-Hexenoate | 100 µM |
| beta-Resorcylic acid | 100 µM |
| 2-sec-butylcyclohexanone | 100 µM |
| Dibutyl sebacate | 100 µM |
| 4-Methyl-5-Vinylthiazole | 100 µM |
| Tridecanoic acid | 100 µM |
| Isopentyl formate | 100 µM |
| 1-phenyl 1-propanol | 100 µM |
| 4-Methoxybenzyl Formate | 100 µM |
| 2-Methyl-3-Furanthiol Acetate | 100 µM |
| 2-Methyltetrahydro-3-furanone | 100 µM |
| Citronellyl Formate | 100 µM |
| Ethyl oleate | 100 µM |
| EDTA | 100 µM |
| 2-(1-propoxyethoxy)ethylbenzene | 100 µM |
| Ethyl 5-Hexenoate | 100 µM |
| trans,trans-2,4-Decadienal | 100 µM |
| 2-Methoxybenzaldehyde | 100 µM |
| Dextrin from potato starch | 100 µM |
| Tetrahydrofurfuryl acetate | 100 µM |
| Oleic acid | 100 µM |
| Farnesylacetone | 100 µM |
| 2-Ethyl-3-methoxypyrazine | 100 µM |
| cis-3-Octen-1-ol | 100 µM |
| (1R)-6,6-Dimethylbicyclo[3.1.1]hept-2-ene-2-methyl acetate | 100 µM |
| Hexadecyl Lactate | 100 µM |
| Methyl 3-(methylthio)propionate | 100 µM |
| methyl 2-(acetylamino)benzoate | 100 µM |
| 3-Methylthio-2-butanone | 100 µM |
| Ethyl trans-4-decenoate | 100 µM |
| Propionic acid trans-2-hexen-1-yl ester | 100 µM |
| Citral dimethyl acetal, mixture of cis and trans | 100 µM |
| Methyl nicotinate | 100 µM |
| 2-Isopropenyl-5-Methyl-5-Vinyltetrahydrofuran | 100 µM |
| 2'-Hydroxyacetophenone | 100 µM |
| 2-Methoxybenzoic Acid | 100 µM |
| 4-Methoxyphenylacetone | 100 µM |
| Ferulic acid | 100 µM |
| Methyl 2-Methylpentanoate | 100 µM |
| Quinine hydrochloride dihydrate | 100 µM |
| sec-Butyl disulfide | 100 µM |
| Isobutyl Hexanoate | 100 µM |
| 2-Methyl-3-furanthiol | 100 µM |
| Allyl Caprylate | 100 µM |
| 2-Acetylthiazole | 100 µM |
| | |

| **BLOCK BOTH MENTHOL AND H2O2 ON TRPA1** | Dose |
|---|---|
| | |
| 2-Isobutyl-3-Methoxypyrazine | 100 µM |
| Piperazine | 100 µM |
| Allyl Cyclohexanepropionate | 100 µM |
| Furfuryl Alcohol | 100 µM |
| **Block menthol, H2O2, and AITC on TRPA1** | 100 µM |
| | |
| Manganese Chloride | 100 µM |
| Phloretin | 100 µM |
| 1-Stearoyl-rac-glycerol | 100 µM |
| Copper(I) iodide | 100 µM |
| 2-Methyl-1,3-Dithiolane | 100 µM |
| Butyl 4-hydroxybenzoate | 100 µM |
| 2,5-Dimethyl-1,4-Dithiane-2,5-Diol | 100 µM |
| Phenylacetaldehyde | 100 µM |
| 2,3-Butanedithiol | 100 µM |
| 2-Pyrazinyl ethanethiol | 100 µM |
| 2-Chloroacetophenone | 100 µM |
| 2-Ethylbenzenethiol | 100 µM |
| 2-Undecanone | 100 µM |
| Diphenyl Disulfide | 100 µM |
| | |
| **Block both H2O2 and AITC on TRPA1** | 100 µM |
| | |
| Ethyl acetoacetate ethylene ketal | 100 µM |
| 1,2-Propanedithiol | 100 µM |
| Isobutyric Acid Isopropyl Ester | 100 µM |
| 2-hydroxy-4- Methylbenzaldehyde | 100 µM |
| Benzyl cinnamate | 100 µM |
| Phenyl salicylate | 100 µM |
| Benzothiazole | 100 µM |
| Ethyl acetoacetate | 100 µM |
| Cinnamon Bark Oil | 100 µM |
| Anisyl Butyrate | 400 µM |
| trans, trans-2,4-Nonadienal | 400 µM |
| 4-Allyl-2,6-dimethoxyphenol | 400 µM |
| o-Methoxycinnamaldehyde | 400 µM |
| 4-Methyl-2-phenyl-2 Pentenal (mixture of cis and trans isomers) | 400 µM |
| | |

| **BLOCK BOTH MENTHOL AND AITC ON TRPA1** | Dose |
|---|---|
| | |
| Zinc Gluconate | 100 µM |
| Erucin | 100 µM |
| Succinic acid | 100 µM |
| delta-Octanolactone | 100 µM |
| 2,5-Dimethylfuran-3-thiol | 100 µM |
| 3-(Methylthio)propyl Isothiocyanate | 100 µM |
| 2-Methyl-3-furanthiol | 100 µM |

### EXAMPLE 10

In an attempt to demonstrate the selective reduction in hydrogen peroxide activation of the TRPV1 receptor, antagonists were tested to determine if they failed to significantly reduce the activation of the TRPV1 receptor by capsaicin (TRPV1 agonist), but yet still reduced TRPV1 receptor activation by hydrogen peroxide. Intracellular Ca⁺⁺ levels were measured using cell based assays as described for EXAMPLES 1, 4, and 5.

**TABLE 16**

| Dose | Name | Ca⁺⁺ counts for activation of TRPV1 by (500 uM H2O2) | Ca⁺⁺ counts | % inhibition of TRPV1 activation by 500 uM H2O2 | Ca⁺⁺ counts for activation of TRPV1 by (350 nM) Capsaicin | Ca⁺⁺ counts | % inhibition of TRPV1 activation by 350 nM Capsaicin |
|---|---|---|---|---|---|---|---|
| 100 µM | *(-)-Bornyl Acetate | 1867 | 877 | 53 | 15029 | 13976 | 7 |
| 100 µM | Hydroxycitronellal | 1650 | 627 | 62 | 15029 | 14728 | 2 |
| 100 µM | Apritone | 1673 | 66 | 96 | 15029 | 13526 | 10 |
| 100 µM | Methyl N,N-Dimethylanthranilate | 1129 | 451 | 60 | 15029 | 15329 | -2 |
| 100 µM | 2-Ethoxy-3-ethylpyrazine | 1129 | 474 | 58 | 15029 | 14878 | 1 |
| 100 µM | L-Piperiton | 1129 | 361 | 58 | 15029 | 13225 | 12 |
| 100 µM | *** Isobornyl Isobutyrate | 1129 | 541 | 52 | 15029 | 14803 | 1.5 |
| 100 µM | ***4-Acetoxy-2,5-dimethyl-3(2H)-furanone | 1129 | 451 | 60 | 15029 | 14578 | 3 |
| 400 µM | Tripropylamine | 1935 | 715 | 63 | 11872 | 11278 | -5 |
| 400 µM | Dihydrojasmone | 1935 | 38 | 98 | 11872 | 11397 | 4 |
| 400 µM | *** 1-Methyl-2-pyrole carboxaldehyde | 1811 | 380 | 79 | 11872 | 11516 | 3 |
| 400 µM | 3-Octyl Acetate | 1811 | 181 | 90 | 11872 | 12109 | -2 |
| 400 µM | 2-Methylbutyl isovalerate | 1811 | 380 | 79 | 11872 | 11278 | -5 |
| 400 µM | Jasminone B | 1844 | 903 | 51 | 11872 | 11635 | 2 |
| 400 µM | ** Piperonyl Isobutyrate | 1844 | 682 | 63 | 11872 | 10804 | 9 |
| 400 µM | * Phenoxyethyl Propionate | 1844 | 737 | 60 | 11872 | 10922 | 8 |
| 400 µM | Vanillin Propylene Glycol Acetate | 1844 | 922 | 50 | 11872 | 11516 | 3 |
| 400 µM | Octenyl Cyclopentanone | 1844 | 922 | 50 | 11872 | 9378 | 1 |
| 400 µM | Butyl Isobutyrate | 1844 | 774 | 58 | 11872 | 11278 | 0.5 |
| 400 µM | *Guaiacwood Oil | 1844 | 737 | 60 | 11872 | 10922 | 8 |
| 400 µM | Tetrahydro-4-methyl-2-(2-methyl-1-propenyl)-2H pyran | 1844 | 866 | 53 | 11872 | 11041 | 7 |
| * | Also TRPA1 agonist | | | | | | |
| ** | TRPA1 enhancer | | | | | | |
| *** | Also reduced H2O2 activation by TRPA1 and TRPA1V1 | | | | | | |

TABLE 16 shows compounds that reduce hydrogen peroxide activation of TRPV1, but do not reduce capsaicin activation of TRPV1. The compounds (Bornyl Acetate, Phenoxyethyl Propionate, Vanillin Propylene Glycol Acetate, and Guaiacwood Oil) are TRPA1 agonists, yet reduce hydrogen peroxide activation of TRPV1. The compound (Piperonyl Isobutyrate) is an enhancer of TRPA1, yet reduces hydrogen peroxide activation of TRPV1. Further, compounds (Isobornyl Isobutyrate, 4-Acetoxy-2,5-dimethyl-3(2H)-furanone, 1-Methyl-2-pyrole carboxaldehyde) reduced hydrogen peroxide activation of TRPA1, in addition to hydrogen peroxide reduction of TRPV1.

For EXAMPLES 11 and 12 a 0.1% sodium lauryl sulfate (SLS) aqueous solution was prepared to contain either 1 ppm or 10 ppm of 1-propanethiol. SLS solution was added to the sample and control solutions to assist in solubilization of the more hydrophobic compounds. Control solutions contained surfactant and 1-propanethiol, while sample solutions contained these components plus a protectant compound added at either 0.01% or 0.05%. 100 µL aliquots of control or test solutions were aliquotted into a 22 mL headspace vial, vortexed for 30 seconds, and incubated for 30 minutes at 60 C in an oven. After that period, 1 mL of head space was sampled and injected into an Agilent 7890 gas chromatograph equipped with a sulfur chemiluminescence detector (GC-SCD). Peak areas of the 1-propanethiol control sample (no protectant compound added) were established by triplicate injection of the control sample. The area of the propanethiol peak produced from samples containing selected protectant molecules were then determined and ratioed to the 1-propanethiol control peak area, subtracted from 1, and multiplied by 100 to calculate percent reduction of the thiol peak, thus indicating the effectiveness of each protectant at reducing the level of thiol.

### EXAMPLE 11

The samples in TABLE 17 were prepared by combining 1 ppm 1-propanethiol with 0.1% sodium lauryl sulfate with 1% of the test compounds. The volatile sulfurs were measured with headspace GC/MS using a SPEME column to capture the volatile sulfur. The results in TABLE 17 below show the efficacy of the dihydrojasmone on the initial screen, as well as the effectiveness of other cyclopentenones for their effectiveness in the reduction of propyl mercaptan.

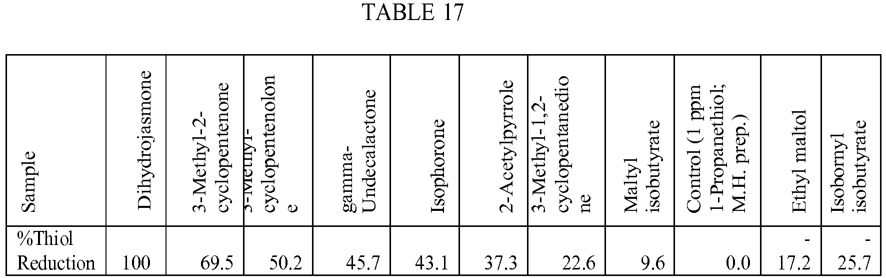

The results from TABLE 17 demonstrate most compounds tested reduced sulfur production, but Dihydrojasmone significantly or completely reduced sulfur production.

### EXAMPLE 12

The samples in TABLE 18 were prepared by combining either 1 or 10 ppm 1-propanethiol with 0.1% sodium lauryl sulfate with either 0.01% or 0.05% of the test compounds. The volatile sulfurs were measured with headspace GC/MS using a SPEME column to capture the volatile sulfur.

**TABLE 18**

| Sample | 1-Propanethiol Concentration | 1-Propanethiol Peak Area | Percent Thiol Reduction vs. Control | Propyl disulfide Peak Area |
|---|---|---|---|---|
| Blank (0.1% SLS) | na | 0.0 | na | 0.0 |
| 1 ppm 1-Propanethiol in 0.1% SLS | 1 ppm | 2,555.3 | na | 1,039.5 |
| 1 ppm 1-Propanethiol in 0.1% SLS | 1 ppm | 2,848.0 | na | 1,043.8 |
| 1 ppm 1-Propanethiol in 0.1% SLS | 1 ppm | 2,970.9 | na | 1,021.8 |
| | *Average* | 2,791.4 | na | na |
| | *Std Dev.* | 213.5 | na | na |
| | % *RSD* | 7.6 | na | na |
| 0.01% Cinnamic aldehyde | 1 ppm | 1,764.0 | 36.8 | 719.2 |
| 0.05% Cinnamic aldehyde | 1 ppm | 1,328.5 | 52.4 | 763.3 |
| 0.01% Anisaldehyde | 1 ppm | 1,354.7, | 60.8 | 607.7 |
| 0.05% Anisaldehyde | 1 ppm | 1,094.5 | 36.1 | 802.3 |
| 0.01% cis-Jasmone | 1 ppm | 1,783.7 | 55.3 | 1,034.2 |
| 0.05% cis-Jasmone | 1 ppm | 1,246.5 | 79.0 | 1,220.2 |
| 0.01% Dihydrojasmone | 1 ppm | 587.4 | 92.1 | 1,633.1 |
| 0.05% Dihydrojasmone | 1 ppm | 221.4 | 64.3 | 1,836.1 |
| 0.01% Methyl jasmonate | 1 ppm | 996.3 | 72.3 | 675.5 |
| 0.05% Methyl jasmonate | 1 ppm | 773.2 | 77.7 | 800.7 |
| 0.01% delta-Damascone | 1 ppm | 623.2 | 82.3 | 344.3 |
| 0.05% delta-Damascone | 1 ppm | 495.2 | na | 1,564.1 |
| 10 ppm 1-Propanethiol in 0.1% SLS | 10 ppm | 28,575.3 | na | 23,521.0 |
| 10 ppm 1-Propanethiol in 0.1% SLS | 10 ppm | 26,691.8 | na | 22,177.3 |
| 10 ppm 1-Propanethiol in 0.1% SLS | 10 ppm | 27,628.9 | na | 22,738.4 |
| | *Average* | 27,632.0 | na | na |
| | *Std Dev.* | 941.8 | na | na |
| | % *RSD* | 3.4 | na | na |
| Blank (0.1% SLS) | na | 107.5 | na | 166.9 |
| 0.01% Cinnamic aldehyde | 10 ppm | 30,403.7 | -10.0 | 6,936.6 |
| 0.05% Cinnamic aldehyde | 10 ppm | 29,201.1 | -5.7 | 8,281.6 |
| 0.01% Anisaldehyde | 10 ppm | 25,575.0 | 8.5 | 10,477.5 |
| 0.05% Anisaldehyde | 10 ppm | 16,178.2 | 41.5 | 4,387.2 |
| 0.01% cis-Jasmone | 10 ppm | 20,614.0 | 25.4 | 15,135.5 |
| 0.05% cis-Jasmone | 10 ppm | 25,378.8 | 8.2 | 22,392.7 |
| 0.01% Dihydrojasmone | 10 ppm | 16,233.8 | 41.3 | 16,939.8 |
| 0.05% Dihydrojasmone | 10 ppm | 9,210.2 | 66.7 | 24,082.8 |
| 0.01% Methyl jasmonate | 10 ppm | 23,259.5 | 15.8 | 15,226.2 |
| 0.05% Methyl jasmonate | 10 ppm | 12,831.5 | 53.6 | 8,446.4 |
| 0.01% delta-Damascone | 10 ppm | 5,252.7 | 81.0 | 5,400.0 |
| 0.05% delta-Damascone | 10 ppm | 5,193.0 | 81.2 | 3,839.7 |

The screening in TABLE 18 below shows the equivalence of dihydrojasmone to delta damascone. The results showed the comparison of low and high levels of propane thiol and how the Michael Acceptors reduce its levels. At the higher thiol levels (10 ppm), delta damascone had higher activity at its lower concentration (0.01%) than any of the other michael acceptor's. Dihydrojasmone at its higher concentration (0.05%) was close to delta damascone's thiol reduction. At the lower levels of thiol (1 ppm), the levels of dihydrojasmone tested showed better reduction in thiol than delta damascone. This data illustrated the need to range find on levels to suit the desired application, as the more sulfur that is present, the more of the Michael Acceptor that is needed, hence the two concentrations of Michael Acceptor tested (0.01% and 0.05%).

### EXAMPLE 13

As shown in TABLE 19 Quantitative structure-activity relationship models (QSAR models) were used to find molecular structures built off a target compound to identify new structures that are predicted to be more efficacious Michael Acceptors based on the data the tested structures. The molecular structure was generated in the computer software Discovery Studio (Accelrys Inc., San Diego, CA), followed by descriptor generation in the computer software CAChe (Developed by Cache Group, Beaverton, OR. Cache and Discovery Studio software were run on a HP 8540w Laptop Computer. All descriptors including logP, Balaban J Index reversed, nucleophilic, electrophilic and radical susceptibility descriptors were calculated in the CAChe software. The electrophilic, nucleophilic and radical susceptibility descriptors estimate how vulnerable a molecule is to an attack by either electrophiles, nucleophiles, or radicals respectively and exact methods of computation are documented in the CAChe User Guides and Quick Start manuals. All default values in the program for both CAChe and Display Studio were used for the computations."

**TABLE 19**

| QSAR Properties | Balaban J Index Reversed | highest electrophilic susceptibility | highest nucleophilic susceptibility | highest radical susceptibility | LogP |
|---|---|---|---|---|---|
| Cinnamic_aldehyde | 1.1361 | 0.3751 | 0.4736 | 0.2807 | 1.949 |
| Anisaldehyde | 1.1177 | 0.4827 | 0.4937 | 0.3415 | 1.573 |
| cis-Jasmone | 1.5372 | 0.4793 | 0.6395 | 0.3973 | 3.108 |
| Dihydrojasmone | 1.6259 | 0.6129 | 0.6533 | 0.5226 | 3.552 |
| Methyl_jasmonate | 1.3367 | 0.6458 | 0.7053 | 0.3528 | 2.356 |
| delta-Damascone | 1.7559 | 0.6149 | 0.8042 | 0.411 | 3.387 |

The QSAR computed properties in TABLE 19 indicate that the highest electrophilic susceptibility most directly separated these three from the others. Other influencers were LogP and highest nucleophilic susceptibility. Indicating that the electrophilic susceptibility, LogP, and nucleophilic susceptibility are the values that best describe the current set of molecules and could be used to create new structures.

### EXAMPLE 14

TABLES 20 and 21 outline shave prep compositions and methods of making. The water soluble polymers (polyethylene oxide, hydroxyethylcellulose) are added to water and mixed until the polymers are completely dissolved (about 30 min.). The aqueous mixture is then heated and the glyceryl oleate, sorbitol and fatty acids are added at about 60 deg. C. and well mixed while the heating continues. At 80-85 deg. C. the triethanolamine is added and mixed for about 20 minutes to form the aqueous soap phase. After cooling the aqueous soap phase to room temperature, the remaining components (i.e., Lubrajel, glycerin, fragrance, colorant, botanicals) are added to the aqueous soap phase and mixed well to form the gel concentrate. (Water may be added if required to bring the batch weight to 100%, thereby compensating for any water loss due to evaporation.) The concentrate is then combined with the volatile post-foaming agent under pressure within the filling line and filled into bottom-gassed aerosol cans with shearing through the valve under nitrogen pressure. Note, Iso E Super can be added the same time as the fragrance.

**TABLE 20**

| | Samples | | | | |
|---|---|---|---|---|---|
| Ingredient | 1 | 2 | 3 | 4 | 5 |
| Sorbitol 70% Solution | 0.97% | 0.97% | 0.97% | 0.97% | 0.97% |
| Glycerin | 0.49% | | 0.49% | | 0.49% |
| Water | QS | QS | QS | QS | QS |
| hydroxyethyl cellulose¹⁸ | 0.49% | 0.49% | 0.49% | 0.49% | 0.49% |
| PEG-90M¹⁹ | 0.06% | 0.06% | 0.06% | 0.06% | 0.06% |
| PEG-23M²⁰ | 0.05% | 0.05% | 0.05% | 0.05% | 0.05% |
| PTFE²¹ | 0.15% | 0.15% | 0.15% | 0.15% | 0.15% |
| Palmitic acid | 7.53% | 7.53% | 7.53% | 7.53% | 7.53% |
| Stearic Acid | 2.53% | 2.53% | 2.53% | 2.53% | 2.53% |
| Glyceryl Oleate | 1.94% | 1.94% | 1.94% | 1.94% | 1.94% |
| Triethanolamine (99%) | 5.88% | 5.88% | 5.88% | 5.88% | 5.88% |
| Lubrajel Oil" | 0.49% | 0.97% | 0.49% | 0.97% | 0.49% |
| Apritone | 0.05% | 0.05% | | | |
| Isobornyisolbutyrate | | 0.05% | 0.05% | | |
| Phloretin | | | | | 0.1% |
| Dihydroj asmone | | | | 0.05% | |
| Menthol | 0.15 | 0.2% | 0.15% | 0.2% | 0.25% |
| Fragrance | 0.87% | 0.87% | 0.87% | 0.87% | 0.87% |
| Other (e.g. Vit E, Aloe, etc.) | 0.10% | 0.10% | 0.10% | 0.10% | 0.10% |
| Dye | 0.10% | 0.10% | 0.10% | 0.10% | 0.10% |
| Isopentane (and) Isobutane | 2.8500% | 2.8500% | 2.8500% | 2.8500% | 2.8500% |

| | | | | | |
|---|---|---|---|---|---|
| ¹⁸ Available as Natrosol 250 HHR from Hercules Inc., Wilmington, DE ¹⁹ Available as Polyox WSR-301 from Amerchol Corp., Piscataway, NJ ²⁰ Available as Polyox WSR N-12K from Amerchol Corp., Piscataway, NJ ²¹ Available as Microslip 519 from Micro Powders Inc., Tarrytown, NY ²² Available from Guardian Laboratories, Hauppauge, NY *QS refers to the term *quantum sufficit,* meaning as much as suffices, where the remainder of the formula hole is filled with this substance | | | | | |

The pre-shave prep samples shown in TABLE 21 are made by weighing out the water in a vessel sufficient to hold the entire batch. Insert an overhead mixer with impeller into the vessel and increase agitation to create a vortex. Pre-blend the thickener and polymer powders. Sprinkle the polymer blend into the vortex until incorporated. Begin heating batch to 70C to hydrate the polymers. Once the batch is at 70C, add the oil and mix until uniform and dispersed. Add the liquid dispersion polymer to the batch and mix until uniform and hydrated, increasing rpms to maintain good mixing. Add the surfactant and mix until uniform and dispersed. Begin cooling batch to below 45C. Once below 45C, add the perfume, preservatives and other temperature-sensitive additives. Cool to below 35C and QS with water. Iso E Super can be added after the sample is cooled to 35 C or along with the perfume.

**TABLE 21**

| | Samples | | | | |
|---|---|---|---|---|---|
| Ingredient | 1 | 2 | 3 | 4 | 5 |
| Water | QS | QS | QS s | QS s | QS s |
| Sepigel 305 (Polyacrylamide & C13-14 Isoparaffin & Laureth-7) | 2.00 | 2.00 | 2.00 | 2.00 | 2.00 |
| Polyox N12K (PEG-23M) | 0.50 | 0.50 | 0.50 | 0.50 | 0.50 |
| Natrosol 250 HHR (HEC) | 0.80 | 0.80 | 0.80 | 0.80 | 0.80 |
| Glycerin 99.7% Usp/Fcc | 5.00 | 5.00 | 5.00 | 5.00 | 5.00 |
| Brij 35 (Laureth-23) | 2.00 | 2.00 | 2.00 | 2.00 | 2.00 |
| | | | | | |
| Disodium EDTA | 0.10 | 0.10 | 0.10 | 0.10 | 0.10 |
| Perfume | 0.15 | 0.15 | 0.15 | 0.15 | 0.15 |
| Glydant Plus | 0.20 | 0.20 | 0.20 | 0.20 | 0.20 |
| Apritone | 0.05% | 0.05% | | | |
| Isobornyisolbutyrate | | 0.05% | 0.05% | | |
| Phloretin | | | | | 0.1% |
| Dihydrojasmone | | | | 0.05% | |
| Menthol | 0.00 | 0.05 | 0.05 | 0.04 | 0.02 |

### EXAMPLE 15

TABLE 22 illustrates shampoo compositions containing menthol and burn sensation blockers (TRPA1/TRPV1 antagonists). The shampoo compositions may be made by mixing the ingredients together at either room temperature or at elevated temperature, e.g., about 72°C. Heat only needs to be used if solid ingredients are to be incorporated into the composition. The ingredients are mixed at the batch processing temperature. Additional ingredients, including electrolytes, polymers, fragrance, menthol, and particles, may be added to the product at room temperature.

**TABLE 22**

| | Samples | | | | | | |
|---|---|---|---|---|---|---|---|
| Ingredient | 1 | 2 | 3 | 4 | 5 | 6 | 7 |
| Water | QS | QS | QS | QS | QS | QS | QS |
| Polyquaterium 76¹ | 0.25 | -- | - | | 0.01 | | |
| Guar, Hydroxylpropyl Trimonium Chloride² | -- | 0.25 | -- | | 0.3 | 0.4 | 0.5 |
| Guar, Hydroxylpropyl Trimonium Chloride³ | | | | 0.25 | | | |
| Polyquaterium 6⁴ | - | - | 0.25 | | | | |
| Sodium Laureth Sulfate (SLE3S)⁵ | 6 | 10.5 | 6 | | 6.0 | | 10.0 |
| Sodium Laureth Sulfate (SLE1S)⁶ | | | | 10.5 | | 12 | |
| Sodium Lauryl Sulfate (SLS)⁷ | 6 | 1.5 | 6 | 1.5 | 7.0 | | 6 |
| Silicone⁸ | 0.75 | 1.00 | 0.5 | 1.00 | | | 1.00 |
| Gel Network ⁹ | | | | 27.3 | | | |
| Cocoamidopropyl Betaine ¹⁰ | 1.0 | 1.0 | 1.0 | 2.00 | 1.0 | 1.0 | |
| Cocoamide MEA ¹¹ | 0.85 | 0.85 | 0.85 | 0.85 | | 1.0 | |
| Ethylene Glycol Distearate¹² | 1.50 | 1.50 | 1.50 | 1.50 | 2.5 | 1.5 | 1.5 |
| Zinc Pyrithione¹³ | | | | | 1.0 | 1.0 | 1.0 |
| Zinc Carbonate ¹⁴ | | | | | 1.6 | 1.6 | 1.6 |
| Sodium Benzoate | 0.25 | 0.25 | 0.25 | 0.25 | 0.25 | 0.25 | 0.25 |
| Disodium EDTA | 0.13 | 0.13 | 0.13 | 0.13 | | | |
| 5-Chloro-2-methyl-4-isothiazolin-3-one, Kathon CG | 0.0005 | 0.0005 | 0.0005 | 0.0005 | 0.0005 | 0.0005 | 0.0005 |
| Sodium Chloride/ Ammonium Xylene Sulfonate | Visc. QS | Visc. QS | Visc. QS | Visc. QS | Visc. QS | Visc. QS | Visc. QS |
| Citric Acid/ Sodium Citrate Dihydrate | pH QS | pH QS | pH QS | pH QS | | | |
| Hydrochloric Acid 6N solution | | | | | pH to 7 | pH to 7 | pH to 7 |
| Menthol | 0.3 | 0.5 | 0.2 | 0.4 | 0.3 | 0.5 | 0.4 |
| Fragrance | 0.7 | 0.7 | 0.7 | 0.7 | 0.7 | 0.8 | 0.9 |

| Ingredient | Wt. % | | | | | | |
|---|---|---|---|---|---|---|---|
| Water | 86.14% | | | | | | |
| Cetyl Alcohol | 3.46% | | | | | | |
| Steary Alcohol | 6.44% | | | | | | |
| Sodium laureth-3 sulfate (28% Active) | 3.93% | | | | | | |
| 5-Chloro-2-methyl-4-isothiazolin -3-one, Kathon CG | 0.03% | | | | | | |

| | | | | | | | |
|---|---|---|---|---|---|---|---|
| 1 Mirapol AT-1, Copolymer of Acrylamide(AM) and TRIQUAT, MW=1,000,000; CD= 1.6 meq./gram; Supplier Rhodia 2 Jaguar C500, MW - 500,000, CD=0.7, supplier Rhodia 3 Jaguar C17 available from Rhodia 4 Mirapol 100S, supplier Rhodia 5 Sodium Laureth Sulfate, supplier P&G 6 Sodium Laureth Sulfate, supplier P&G 7 Sodium Lauryl Sulfate, supplier P&G 8 Dimethicone Fluid, Viscasil 330M; 30 micron particle size; supplier Momentive Silicones 9 Gel Networks; See Composition below. The water is heated to about 74°C and the Cetyl Alcohol, Stearyl Alcohol, and the SLES Surfactant are added to it. After incorporation, this mixture was passed through a heat exchanger where it was cooled to about 35°C. As a result of this cooling step, the Fatty Alcohols and surfactant crystallized to form a crystalline gel network. 10 Tegobetaine F-B, supplier Evonik 11 Monamid CMA, supplier Evonik 12 Ethylene Glycol Distearate, EGDS Pure, supplier Evonik 13 ZPT from Arch Chemical 14 Zinc carbonate from the Bruggeman Group | | | | | | | |

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

## Claims

1. A personal care composition comprising:
a) at least 0.5% by weight of the personal care composition menthol and
b) at least one of an antagonist to TRPV1 receptor, wherein the TRPV1 antagonist is at least one of copper (I) iodide; butyl 4-hydroxybenzoate; diphenyl disulfide; 2,3-butanedithiol; 2,5-dimethyl-1.4-dithiane-2,5-diol; cellulose acetate.

2. The personal care composition of claim 1, wherein the TRPV1 antagonist is present in an amount of from 0.0001% to 0.2%, by weight of the personal care composition.

3. The personal care composition of claim 1, wherein the TRPV1 antagonist is present in an amount of from 0.001% to 0.1%.

4. A method of reducing the negative sensations produced by the application of personal care compositions comprising:
a) providing a personal care composition according to claim 1;
b) contacting a body surface with the personal care composition.

5. The method of claim 4, wherein at 5 minutes after contacting the body surface with the personal care composition negative sensations are reduced by about 40%.

6. The method of any of claims 4 to 5, wherein the personal care composition further comprises a TRPA1 antagonist.

## Patentansprüche

1. Körperpflegezusammensetzung, umfassend:
a) mindestens 0,5 Gew.-% der Körperpflegezusammensetzung Menthol und
b) mindestens einen von einem Antagonisten zu TRPV1 -Rezeptor, wobei der TRPV1-Antagonist mindestens eines ist von Kupfer(I)-iodid; Butyl-4-hydroxybenzoat; Diphenyldisulfid; 2,3-Butandithiol; 2,5-Dimethyl-1,4-dithian-2,5-diol; Celluloseacetat;

2. Körperpflegezusammensetzung nach Anspruch 1, wobei der TRPV1 - Antagonist in einer Menge von 0,0001 Gew.-% bis 0,2 Gew.-% der Körperpflegezusammensetzung vorhanden ist.

3. Körperpflegezusammensetzung nach Anspruch 1, wobei der TRPV1 - Antagonist in einer Menge von 0,001 % bis 0,1 % vorhanden ist.

4. Verfahren zum Reduzieren der negativen Empfindungen, die durch die Anwendung von Körperpflegezusammensetzungen erzeugt werden, umfassend:
a) Bereitstellen einer Körperpflegezusammensetzung nach Anspruch 1;
b) Inkontaktbringen einer Körperoberfläche mit der Körperpflegezusammensetzung.

5. Verfahren nach Anspruch 4, wobei die negativen Empfindungen bei 5 Minuten nach dem Inkontaktbringen der Körperoberfläche mit der Körperpflegezusammensetzung um etwa 40 % reduziert werden.

6. Verfahren nach einem der Ansprüche 4 bis 5, wobei die Körperpflegezusammensetzung ferner einen TRPA1-Antagonisten umfasst.

## Revendications

1. Composition de soins personnels comprenant :
a) au moins 0,5 % en poids de la composition de soins personnels de menthol et
b) au moins l'un parmi un antagoniste vis-à-vis du récepteur de TRPV1, l'antagoniste de TRPV1 étant au moins l'un parmi les iodure de cuivre (I) ; 4-hydroxybenzoate de butyle ; disulfure de diphényle ; 2,3 butanedithiol ; 2,5-diméthyl-1,4-dithiane-2,5-diol ; acétate de cellulose.

2. Composition de soins personnels selon la revendication 1, dans laquelle l'antagoniste de TRPV1 est présent en une quantité allant de 0,0001 % à 0,2 %, en poids de la composition de soins personnels.

3. Composition de soins personnels selon la revendication 1, dans laquelle l'antagoniste de TRPV1 est présent en une quantité allant de 0,001 % à 0,1 %.

4. Procédé de réduction des sensations négatives produites par l'application de compositions de soins personnels comprenant :
a) fournir une composition de soins personnels selon la revendication 1 ;
b) mettre en contact une surface corporelle avec la composition de soins personnels.

5. Procédé selon la revendication 4, dans lequel, 5 minutes après la mise en contact de la surface corporelle avec la composition de soins personnels, les sensations négatives sont réduites d'environ 40 %.

6. Procédé selon l'une quelconque des revendications 4 à 5, dans lequel la composition de soins personnels comprend en outre un antagoniste de TRPA1.
